# EUROPEAN PATENT SPECIFICATION

(11) **EP 2 873 727 B1**
(45) Date of publication and mention of the grant of the patent: **06.11.2019**
(21) Application number: 13816708.5
(22) Date of filing: 12.07.2013
(51) Int. Cl.: C12N 5/10, A61K 35/12, A61P 1/16, A61P 3/00, A61P 3/04, A61P 3/06, A61P 3/10, A61P 9/10, A61P 9/12, A61P 19/06, C12N 5/077, C12N 15/09, A61K 48/00

(54) **BROWN FAT CELLS AND METHOD FOR PREPARING SAME**
BRAUNE FETTZELLEN UND VERFAHREN ZUR HERSTELLUNG DAVON
CELLULES ADIPEUSES BRUNES ET LEUR PROCÉDÉ DE PRÉPARATION

(30) Priority: 12.07.2012 JP 2012156066
(43) Date of publication of application: 20.05.2015
(73) Proprietor: Kyoto Prefectural Public University Corporation, Kyoto-shi, Kyoto 602-8566 (JP)
(72) Inventor: KISHIDA, Tsunao, Kyoto-shi Kyoto 602-8566 (JP); MAZDA, Osam, Kyoto-shi Kyoto 602-8566 (JP)
(74) Representative: Held, Stephan
(86) International application number: PCT/JP2013/069226
(87) International publication number: WO 2014/010746

(56) References cited:
- WO-A1-2010/080985
- WO-A1-2010/080985
- WO-A1-2011/050334
- WO-A1-2011/050334
- WO-A1-2011/102531
- WO-A1-2012/147853
- WO-A1-2012/147853
- SHINGO KAJIMURA ET AL: "Initiation of myoblast to brown fat switch by a PRDM16-C/EBP-[beta] transcriptional complex", NATURE, vol. 460, no. 7259, 27 August 2009 (2009-08-27), pages 1154-1158, XP55055707, ISSN: 0028-0836, DOI: 10.1038/nature08262
- TIM AHFELDT ET AL: "Programming human pluripotent stem cells into white and brown adipocytes", NATURE CELL BIOLOGY, vol. 14, no. 2, 1 January 2012 (2012-01-01), pages 209-219, XP55055708, ISSN: 1465-7392, DOI: 10.1038/ncb2411
- NATHALIE BILLON ET AL: "Developmental Origins of the Adipocyte Lineage: New Insights from Genetics and Genomics Studies", STEM CELL REVIEWS AND REPORTS, HUMANA PRESS INC, NEW YORK, vol. 8, no. 1, 2 March 2011 (2011-03-02), pages 55-66, XP035019898, ISSN: 1558-6804, DOI: 10.1007/S12015-011-9242-X
- JIANGUO ZHU ET AL: "Direct Conversion of Porcine Embryonic Fibroblasts into Adipocytes by Chemical Molecules", CELLULAR REPROGRAMMING, vol. 14, no. 2, 28 February 2012 (2012-02-28), pages 99-105, XP55111928, US ISSN: 2152-4971, DOI: 10.1089/cell.2011.0074
- KAJIMURA SHINGO ET AL.: 'Initiation of myoblast to brown fat switch by a PRDM16-C/ EBP-beta transcriptional complex' NATURE vol. 460, 2009, pages 1154 - 1159, XP055055707
- AHFELDT TIM ET AL.: 'Programming human pluripotent stem cells into white and brown adipocytes' NATURE CELL BIOLOGY vol. 14, no. 2, February 2012, pages 209 - 219, XP055055708
- JIMENEZ-PREITNER MARIA ET AL.: 'Plac8 Is an Inducer of C/EBPbeta Required for Brown Fat Differentiation, Thermoregulation, and Control of Body Weight' CELL METABOLISM vol. 14, 2011, pages 658 - 670, XP028334807

## Description

### Technical Field

The present invention relates to a method for preparing brown adipocytes. The present invention also relates to a preventive or therapeutic agent for obesity, diabetes, impaired glucose tolerance, abnormal lipid metabolism, arteriosclerotic disease, hypertension, hyperuricemia, gout, non-alcoholic fatty liver disease or metabolic syndrome.

### Background Art

Obesity and obesity-related metabolic diseases, for example, diabetes and metabolic syndrome, have become an extremely serious medical and social problem in advanced industrial countries. In adiposis, white adipocytes not only store as fatty acids excess energy derived from food, but also produce various hormones and cytokines to cause impaired glucose tolerance or abnormal lipid metabolism, leading to type II diabetes, arteriosclerotic disease, hypertension, hyperuricemia, gout, non-alcoholic fatty liver disease, or like diseases.

On the other hand, brown adipocytes (BAs) are, unlike white adipocytes, cells that oxidize and degrade fatty acids, and release the energy as heat. This is because uncoupling protein 1 (UCP1), a mitochondrial inner membrane protein specifically expressed in BAs, uncouples oxidative phosphorylation. In rodents including mice, BAs are present between the shoulder blades, at the posterior region of neck, at the mediastinum, around the kidneys, and the like. It is also known from analysis of UCP1 knockout mice etc. that BAs suppress obesity and impaired glucose tolerance.

Regarding humans, until recently, brown adipocytes had been considered to be present only in infants, but nonexistent in adults. In 2009, however, it was revealed that even adults have brown adipocytes in, for example, subcutaneous tissue of the supraclavicular region and around the aorta (Non-patent Literature 1 to 3). The number and function of brown adipocytes vary greatly among individuals, and are inversely correlated with body mass index (BMI) and fasting blood glucose. Many brown adipocytes are present in lean humans, whereas considerable reduction in brown adipocytes is seen in patients with obesity, diabetes, or hyperlipidemia. Thus, brown adipocytes are important in analysis of genetic predisposition to obesity, diabetes, hyperlipidemia, or like diseases, investigation of environmental factors, elucidation of pathological conditions, or development of new diagnostic methods and techniques for determining therapeutic effects or the like. Brown adipocytes are also believed to be extremely useful for development of new therapeutic agents for these diseases. Further, if brown adipocytes can be replenished in patients with obesity, diabetes, hyperlipidemia, metabolic syndrome, or like diseases, there is a possibility that this could be a new treatment method for these diseases.

A method for preparing brown and white adipocytes from human iPS cells via mesenchymal stem cells is known (Non-patent Literature 4); however, the induction of differentiation from iPS cells into brown and white adipocytes requires time until the final adipocytes are obtained, and, because the adipocytes are derived from iPS cells, poses the risk of oncogenesis.

The following are, for example, reported examples of direct conversion of somatic cells:
mouse fibroblasts → chondrocytes (introduction of SOX9, Klf4, and c-Myc genes, Patent Literature 1);
mouse fibroblasts → cardiac muscle cells (introduction of GATA4, Mef2c, and Tbx5 genes);
mouse fibroblasts → hepatocytes (introduction of Hnf4α and (Foxa1, Foxa2, or Foxa3) genes);
mouse fibroblasts → neural stem cells (for example, introduction of Sox2 and FoxG1 genes); and
mouse, human cells → hematopoietic stem cells.

It has hitherto been known that introduction of PRDM16 and C/EBPβ genes into myoblasts or fibroblasts induces differentiation into "brown adipocyte-like cells" (Patent Literature 2 and Non-patent Literature 5). However, the cells differentiated using PRDM16 and C/EBPβ have unsatisfactory properties as brown adipocytes, such as very low expression level of UCP1.

### Citation List

### Patent Literature

PTL 1: WO2010/071210
PTL 2: WO2010/080985A8

### Non-patent Literature

NPL 1: Saito M. et al., Diabetes 58: 1526, 2009
NPL 2: Cypess A.M. et al., N Eng J Med 360: 1509, 2009
NPL 3: Van Merken Lichtenbelt W.D. et al., N Engl J Med 360: 1500, 2009
NPL 4: Tim Ahfeldt et al., Nature Cell Biology Vol. 14, No. 2, 2012
NPL 5: Kajimura S. et al., Nature 460: 1154, 2009

### Summary of Invention

### Technical Problem

An object of the present invention is to provide a preventive or therapeutic agent for obesity, diabetes, impaired glucose tolerance, abnormal lipid metabolism, arteriosclerotic disease, hypertension, hyperuricemia, gout, non-alcoholic fatty liver disease, or metabolic syndrome; a method for preventing or treating such diseases or conditions; a grafting material that is effective in preventing or treating such diseases or conditions; and a preparation method therefor.

### Solution to Problem

The present invention describes brown adipocytes and a method for preparing the same; a grafting material comprising the brown adipocytes; a preventive or therapeutic agent for various diseases or conditions, the preventive or therapeutic agent comprising the brown adipocytes; and use of the brown adipocytes.
Item 1. A method for preparing a brown adipocyte from a somatic cell of a mammal by introducing at least one brown adipocyte-related gene or expression product thereof and at least one reprogramming-related gene or expression product thereof into the somatic cell, the brown adipocyte-related gene being at least one member selected from the group consisting of PRDM16(P) and C/EBPβ(C), the reprogramming-related gene being at least one member selected from the group consisting of Myc family genes, GLIS family genes, Klf family genes, Oct family genes, Sox family genes, and Lin-28.
Item 2. The method according to Item 1, wherein the somatic cell is a fibroblast or a white adipocyte.
Item 3. The method according to Item 1 or 2, wherein the brown adipocyte-related gene or expression product thereof is C/EBPβ.
Item 4. The method according to Item 1 or 2, wherein the reprogramming-related gene or expression product thereof comprises c-Myc or L-Myc.
Item 5. The method according to Item 1 or 2, wherein the reprogramming-related gene or expression product thereof comprises c-Myc.
Item 6. The method according to Item 1 or 2, wherein a combination of the brown adipocyte-related gene or expression product thereof and the reprogramming-related gene or expression product thereof to be introduced into the somatic cell is any combination selected from the group consisting of PCM, CM, PCL, CL, PCG, CG, PCML, CML, PCM Oct3/4, CM Oct3/4, PCMG, CMG, PCL Oct3/4, CL Oct3/4, PCLG, CLG, PCML Oct3/4, CML Oct3/4, PCMLG, CMLG, PCM Oct3/4 G, CM Oct3/4 G, PCL Oct3/4 G, CL Oct3/4 G, PCML Oct3/4 G, CML Oct3/4 G, wherein P represents PRDM16, C represents C/EBPβ, M represents c-Myc, L represents L-Myc, and G represents Glis1.
Item 7. The method according to Item 6, wherein the combination of the brown adipocyte-related gene or expression product thereof and the reprogramming-related gene or expression product thereof to be introduced into the somatic cell is any combination selected from the group consisting of PCM, CM, PCL, CL, PCML, CML, PCM Oct3/4, CM Oct3/4, PCMG, CMG, PCL Oct3/4, CL Oct3/4, PCLG, CLG, PCML Oct3/4, CML Oct3/4, PCMLG, CMLG, PCM Oct3/4 G, CM Oct3/4 G, PCL Oct3/4 G, CL Oct3/4 G, PCML Oct3/4 G, and CML Oct3/4 G.
Item 8. The method according to Item 6, wherein the combination of the brown adipocyte-related gene or expression product thereof and the reprogramming-related gene or expression product thereof to be introduced into the somatic cell is any combination selected from the group consisting of PCM, CM, PCML, CML, PCMG, CMG, PCLG, CLG, PCMLG, and CMLG.
Item 9. A brown adipocyte derived from a somatic cell of a mammal, the brown adipocyte comprisingat least one brown adipocyte-related gene or expression product thereof and at least one reprogramming-related gene or expression product thereof, the brown adipocyte-related gene being at least one member selected from the group consisting of PRDM16(P) and C/EBPβ(C), the reprogramming-related gene being at least one member selected from the group consisting of Myc family genes, GLIS family genes, Klf family genes, Oct family genes, Sox family genes, and Lin-28.
Item 10. A preventive or therapeutic agent for obesity, diabetes, impaired glucose tolerance, abnormal lipid metabolism, arteriosclerotic disease, hypertension, hyperuricemia, gout, non-alcoholic fatty liver disease, or metabolic syndrome, the preventive or therapeutic agent comprising a brown adipocyte prepared by the method according to any one of Items 1 to 8 or the brown adipocyte according to Item 9 as an active ingredient.
Item 11. Use of a brown adipocyte prepared by the method according to any one of Items 1 to 8 or the brown adipocyte according to Item 9 for the prevention or treatment of obesity, diabetes, impaired glucose tolerance, abnormal lipid metabolism, arteriosclerotic disease, hypertension, hyperuricemia, gout, non-alcoholic fatty liver disease, or metabolic syndrome.
Item 12. A grafting material comprising a brown adipocyte prepared by the method according to any one of Items 1 to 8 or the brown adipocyte according to Item 9.

### Advantageous Effects of Invention

Unlike the above-described prior art techniques, the present invention can efficiently generate brown adipocytes having higher expression of UCP1 and more excellent properties as brown adipocytes by using at least one reprogramming-related gene in addition to PRDM16 and/or C/EBPβ.

Unlike the above-described prior art techniques, the present invention can also efficiently generate brown adipocytes having excellent properties as brown adipocytes by using at least one reprogramming-related gene in addition to C/EBPβ, even when PRDM16 is not used.

Transplanting brown adipocytes in a living body is effective in, for example, the prevention or treatment for obesity, metabolic syndrome, or diseases or conditions related to these, such as diabetes (in particular, type II diabetes), impaired glucose tolerance, abnormal lipid metabolism, arteriosclerotic disease, hypertension, hyperuricemia, gout, and non-alcoholic fatty liver disease, and in removal of visceral fat.

Since brown adipocytes, which burn fat, are also effective in removal of visceral fat and/or subcutaneous fat, injecting brown adipocytes is also effective in beauty treatment such as local removal of fat, decrease in percent of body fat, and removal of subcutaneous fat.

### Brief Description of Drawings

Fig. 1 illustrates the summary of Example 1.
Fig. 2A illustrates results of Oil Red O staining for well Nos. 1 to 12 of the cells obtained in Example 2. aHDFs, day 14.
Fig. 2B illustrates results of Oil Red O staining for well Nos. 13 to 24 of the cells obtained in Example 2. aHDFs, day 14.
Fig. 2C illustrates results of Oil Red O staining for well Nos. 25 to 36 of the cells obtained in Example 2. aHDFs, day 14.
Fig. 2D illustrates results of Oil Red O staining for well Nos. 37 to 48 of the cells obtained in Example 2. aHDFs, day 14.
Fig. 2E illustrates results of Oil Red O staining for well Nos. 49 to 60 of the cells obtained in Example 2. aHDFs, day 14.
Fig. 2F illustrates results of Oil Red O staining for well Nos. 61 to 65 of the cells obtained in Example 2. aHDFs, day 14.
Fig. 3 illustrates the lipid content (vertical axis), OD of Oil Red O extracts (relative values) of well Nos. 1 to 65 of the cells obtained in Example 3. aHDFs, day 14.
Fig. 4 illustrates the UCP1 mRNA levels (relative values) of well Nos. 1 to 65 of the brown adipocytes obtained in Example 4. aHDFs, day 12.
Fig. 5 illustrates relative UCP1 mRNA levels of the cells obtained in Example 5. aHDFs.
Fig. 6 illustrates results of phase contrast, mitochondrial staining, Oil Red O staining of five types of cells (control, PRDM16, C/EBPβ, PRDM16 + C/EBPβ, PRDM16 + C/EBPβ + cMyc). aHDFs.
Fig. 7 illustrates results of Oil Red O staining of cells obtained by treating ADSCs as shown in Fig. 1. ADSCs, day 22.
Fig. 8 illustrates results of quantification of mRNA expression of UCP1, CIDEA, and AdipoQ in cells obtained by treating human adipose-derived stem cells (ADSCs) as shown in Fig. 1. The vertical axis "RQ" indicates relative values of mRNA. ADSCs, day 22.
Fig. 9 illustrates an image of Oil Red O staining , body weight, and rectal temperature obtained when mouse iPS-derived BAs or non-induced cells as a control were transplanted under the abdominal skin of syngeneic mice.
Fig. 10 illustrates results of thermographic images obtained when mouse iPS-derived BAs or non-induced cells as a control were transplanted under the abdominal skin of syngeneic mice. Thermographic visualization shows thermogenesis at the induced BA graft. The body surface temperature went up remarkably in transplanted BA group compared with the control group.
Fig. 11 illustrates body weight change in mice into which iPS-derived BAs were transplanted and non-transplanted mice when the mice were fed a high-calorie diet and a normal diet.
Fig. 12 illustrates results of serum lipids of mice into which iPS-derived BAs were transplanted and non-transplanted mice 4 weeks after transplantation. The mice were fed a high-calorie diet.
Fig. 13 illustrates results of an examination of morphology and expression of stem cell markers in iPS cells established from somatic cells of type II diabetic mice.
Fig. 14 illustrates brown adipocytes induced from iPS cells (top of figure). The brown adipocytes were transplanted into KK-Ay mice, and the results show that the increase in non-fasting blood glucose levels was moderate (lower left) and that urinary glucose was not detected (lower right, three weeks post-transplantation). Progression of diabetes was observed in the controls, i.e., non-transplanted KK-Ay mice and mice into which cells that were not induced into brown adipocytes were transplanted.
Fig. 15 illustrates results of measurement of body weight, and NEFA and neutral fat in the serum in KK-Ay mice into which iPS-derived BAs were transplanted, non-transplanted mice, and GFP control mice.
Fig. 16 illustrates the amount of adiponectin in the serum and the amount of food intake in KK-Ay mice into which iPS-derived BAs were transplanted, mice into which non-induced cells were transplanted, and non-translated control mice.
Fig. 17 illustrates characteristics of brown adipocytes inducedfrom normal human dermal fibroblasts and iPS cells.
Fig. 18 illustrates characteristics of brown adipocytes inducedfrom normal human dermal fibroblasts.
Fig. 19 illustrates characteristics of brown adipocytes inducedfrom normal human dermal fibroblasts.
Fig. 20 illustrates direct reprogramming of normal human dermal fibroblasts into brown adipocytes, using episomal vectors.
Fig. 21 illustrates brown adipocytes inducedfrom mouse embryonic fibroblasts (MEFs).
Fig. 22 illustrates function *in vivo* of brown adipocytes inducedfrom mouse embryonic fibroblasts (MEFs).
Fig. 23 illustrates function *in vivo* of brown adipocytes inducedfrom mouse embryonic fibroblasts (MEFs).

### Description of Embodiments

Examples of diseases to be treated using the brown adipocytes of the present invention as a grafting material include obesity, metabolic syndrome, and diseases or conditions related to these, for example, diabetes (in particular, type II diabetes), impaired glucose tolerance, abnormal lipid metabolism, arteriosclerotic disease, hypertension, hyperuricemia, gout, non-alcoholic fatty liver disease, and the like. The brown adipocytes of the present invention can also be used in beauty applications for removing fat from the abdominal portion, around the jaw, the thighs, or the like. Since administration of brown adipocytes reduces the amount of fat, in particular, white adipocytes, such as visceral fat and subcutaneous fat, and suppresses body weight increase even when a high-calorie diet is consumed, it is useful for both the prevention and treatment of obesity, metabolic syndrome, or diseases or conditions related to these. The present invention can also be used not only for the prevention or treatment of disease, but also for other purposes, such as health promotion and beauty (for example, removal of visceral fat and subcutaneous fat from the abdominal portion, jaw, arms, thighs, or the like). Any treatment provided to humans for health promotion and beauty is also called treatment for reasons of convenience in the present specification. In this case, reference to a "patient" can be deemed to refer to a "healthy person" or a "human," and reference to "disease" can be deemed to refer to "health promotion," "beauty," etc.

The present invention can also be used in the treatment of disease not only for humans, but also for animals kept as pets, such as dogs and cats, and animals kept as livestock, such as cattle, horses, pigs, sheep, and chickens. In this case, reference to a "patient" or a "human" can be deemed to refer to a "diseased animal" or "animal."

The term "grafting material" refers to a material for introducing brown adipocytes into a living body. Brown adipocytes can also be used as a grafting material to be introduced into breasts or the like for beauty treatment. "Grafting material" encompasses a material that is to be transplanted into the same or different individuals after somatic cells are converted to brown adipocytes *in vitro.*

Somatic cells to be used in the method of the present invention are not particularly limited. Examples thereof include fibroblasts, epithelial cells (e.g., skin epidermal cells, oral mucosal epithelial cells, airway mucosal epithelial cells, and intestinal mucosal epithelial cells), epidermal cells, gingival cells (gingival fibroblasts and gingival epithelial cells), dental pulp cells, white adipocytes, subcutaneous adipocytes, visceral adipocytes, muscle cells, blood cells, and the like. Preferable examples include fibroblasts, epidermal cells (keratinocyte), and the like. Examples of somatic cells also include somatic cells prepared from somatic stem cells, such as mesenchymal stem cells (MSCs), neural stem cells, hepatic stem cells, intestinal stem cells, skin stem cells, hair follicle stem cells, and melanocyte stem cells, by induction of differentiation, dedifferentiation, or reprogramming. Examples of somatic cells also include somatic cells prepared by inducing various somatic cells into other cells by induction of differentiation, dedifferentiation, or reprogramming. Examples of somatic cells also include somatic cells prepared from germ line cells by induction of differentiation, dedifferentiation, or reprogramming. Examples of somatic cells also include somatic cells prepared from embryonic stem cells (ES cells) or induced pluripotent stem cells (iPS cells) by induction of differentiation or reprogramming. The "somatic cell" of the present invention also encompasses ES cells, iPS cells, and germ line cells, although they are not, strictly speaking, somatic cells (in this case, reference to "somatic cell" can be deemed to refer to "ES cell," "iPS cell," or "germ line cell"). Examples of somatic cells also include cultured cells and somatic cells prepared from cultured cells by induction of differentiation, dedifferentiation, or reprogramming. These somatic cells may be derived from an adult, an infant, or an embryo. In an important embodiment of the method of the present invention, for example, direct reprogramming is performed by introducing at least one brown adipocyte-related gene or expression product thereof and at least one reprogramming-related gene or expression product thereof into differentiated somatic cells. However, brown adipocytes can also be obtained by the method of the present invention that is direct reprogramming by introducing at least one brown adipocyte-related gene or expression product thereof and at least one reprogramming-related gene or expression product thereof into pluripotent cells, such as ES cells, iPS cells, or other stem cells.

The somatic cells are particularly preferably derived from humans.

In the method of the present invention, combinations of the following genes or expression products thereof are introduced into somatic cells. Here, "expression product" is, for example, mRNA or protein of each gene.

To obtain brown adipocytes, at least one brown adipocyte-related gene or expression product thereof, and at least one reprogramming-related gene or expression product thereof are introduced. The brown adipocyte-related gene is at least one member selected from the group consisting of PRDM16(P) and C/EBPβ(C), and the reprogramming-related gene is at least one member selected from the group consisting of Myc family genes (c-Myc(M), N-Myc, L-Myc(L), S-Myc, and B-Myc), GLIS family genes (GLIS1(G), GLIS 2, and GLIS 3), Klf family genes (KLF1, KLF2, KLF3, KLF4(K), KLF5, KLF6, KLF7, KLF8, KLF9, KLF10, KLF11, KLF12, KLF13, KLF14, KLF15, KLF16, and KLF17), Oct family genes (such as Oct3/4), Sox family genes (such as Sox2), and Lin-28. The reprogramming-related gene preferably comprises c-Myc or L-Myc, and more preferably c-Myc.

Examples of specific combinations of the brown adipocyte-related gene or expression product thereof and the reprogramming-related gene or expression product thereof to be introduced into somatic cells include PCM, PM, CM, PCL, PL, CL, PCK, PK, CK, PCG, PG, CG, PCML, PML, CML, PCMK, PMK, CMK, PCMG, PMG, CMG, PCLK, PLK, CLK, PCLG, PLG, CLG, PCKG, PKG, CKG, PCMLK, PMLK, CMLK, PCMLG, PMLG, CMLG, PCMKG, PMKG, CMKG, PCLKG, PLKG, CLKG, PCMLKG, PMLKG, CMLKG, PCM Lin-28, PM Lin-28, CM Lin-28, PCL Lin-28, PL Lin-28, CL Lin-28, PCK Lin-28, PK Lin-28, CK Lin-28, PCG Lin-28, PG Lin-28, CG Lin-28, PCML Lin-28, PML Lin-28, CML Lin-28, PCMK Lin-28, PMK Lin-28, CMK Lin-28, PCMG Lin-28, PMG Lin-28, CMG Lin-28, PCLK Lin-28, PLK Lin-28, CLK Lin-28, PCLG Lin-28, PLG Lin-28, CLG Lin-28, PCKG Lin-28, PKG Lin-28, CKG Lin-28, PCMLK Lin-28, PMLK Lin-28, CMLK Lin-28, PCMLG Lin-28, PMLG Lin-28, CMLG Lin-28, PCMKG Lin-28, PMKG Lin-28, CMKG Lin-28, PCLKG Lin-28, PLKG Lin-28, CLKG Lin-28, PCMLKG Lin-28, PMLKG Lin-28, CMLKG Lin-28, PCM Oct3/4, PM Oct3/4, CM Oct3/4, PCL Oct3/4, PL Oct3/4, CL Oct3/4, PCK Oct3/4, PK Oct3/4, CK Oct3/4, PCG Oct3/4, PG Oct3/4, CG Oct3/4, PCML Oct3/4, PML Oct3/4, CML Oct3/4, PCMK Oct3/4, PMK Oct3/4, CMK Oct3/4, PCMG Oct3/4, PMG Oct3/4, CMG Oct3/4, PCLK Oct3/4, PLK Oct3/4, CLK Oct3/4, PCLG Oct3/4, PLG Oct3/4, CLG Oct3/4, PCKG Oct3/4, PKG Oct3/4, CKG Oct3/4, PCMLK Oct3/4, PMLK Oct3/4, CMLK Oct3/4, PCMLG Oct3/4, PMLG Oct3/4, CMLG Oct3/4, PCMKG Oct3/4, PMKG Oct3/4, CMKG Oct3/4, PCLKG Oct3/4, PLKG Oct3/4, CLKG Oct3/4, PCMLKG Oct3/4, PMLKG Oct3/4, CMLKG Oct3/4, PCM Sox2, PM Sox2, CM Sox2, PCL Sox2, PL Sox2, CL Sox2, PCK Sox2, PK Sox2, CK Sox2, PCG Sox2, PG Sox2, CG Sox2, PCML Sox2, PML Sox2, CML Sox2, PCMK Sox2, PMK Sox2, CMK Sox2, PCMG Sox2, PMG Sox2, CMG Sox2, PCLK Sox2, PLK Sox2, CLK Sox2, PCLG Sox2, PLG Sox2, CLG Sox2, PCKG Sox2, PKG Sox2, CKG Sox2, PCMLK Sox2, PMLK Sox2, CMLK Sox2, PCMLG Sox2, PMLG Sox2, CMLG Sox2, PCMKG Sox2, PMKG Sox2, CMKG Sox2, PCLKG Sox2, PLKG Sox2, CLKG Sox2, PCMLKG Sox2, PMLKG Sox2, CMLKG Sox2, PCM Lin-28 Oct3/4, PM Lin-28 Oct3/4, CM Lin-28 Oct3/4, PCL Lin-28 Oct3/4, PL Lin-28 Oct3/4, CL Lin-28 Oct3/4, PCK Lin-28 Oct3/4, PK Lin-28 Oct3/4, CK Lin-28 Oct3/4, PCG Lin-28 Oct3/4, PG Lin-28 Oct3/4, CG Lin-28 Oct3/4, PCML Lin-28 Oct3/4, PML Lin-28 Oct3/4, CML Lin-28 Oct3/4, PCMK Lin-28 Oct3/4, PMK Lin-28 Oct3/4, CMK Lin-28 Oct3/4, PCMG Lin-28 Oct3/4, PMG Lin-28 Oct3/4, CMG Lin-28 Oct3/4, PCLK Lin-28 Oct3/4, PLK Lin-28 Oct3/4, CLK Lin-28 Oct3/4, PCLG Lin-28 Oct3/4, PLG Lin-28 Oct3/4, CLG Lin-28 Oct3/4, PCKG Lin-28 Oct3/4, PKG Lin-28 Oct3/4, CKG Lin-28 Oct3/4, PCMLK Lin-28 Oct3/4, PMLK Lin-28 Oct3/4, CMLK Lin-28 Oct3/4, PCMLG Lin-28 Oct3/4, PMLG Lin-28 Oct3/4, CMLG Lin-28 Oct3/4, PCMKG Lin-28 Oct3/4, PMKG Lin-28 Oct3/4, CMKG Lin-28 Oct3/4, PCLKG Lin-28 Oct3/4, PLKG Lin-28 Oct3/4, CLKG Lin-28 Oct3/4, PCMLKG Lin-28 Oct3/4, PMLKG Lin-28 Oct3/4, CMLKG Lin-28 Oct3/4, PCM Oct3/4 Sox2, PM Oct3/4 Sox2, CM Oct3/4 Sox2, PCL Oct3/4 Sox2, PL Oct3/4 Sox2, CL Oct3/4 Sox2, PCK Oct3/4 Sox2, PK Oct3/4 Sox2, CK Oct3/4 Sox2, PCG Oct3/4 Sox2, PG Oct3/4 Sox2, CG Oct3/4 Sox2, PCML Oct3/4 Sox2, PML Oct3/4 Sox2, CML Oct3/4 Sox2, PCMK Oct3/4 Sox2, PMK Oct3/4 Sox2, CMK Oct3/4 Sox2, PCMG Oct3/4 Sox2, PMG Oct3/4 Sox2, CMG Oct3/4 Sox2, PCLK Oct3/4 Sox2, PLK Oct3/4 Sox2, CLK Oct3/4 Sox2, PCLG Oct3/4 Sox2, PLG Oct3/4 Sox2, CLG Oct3/4 Sox2, PCKG Oct3/4 Sox2, PKG Oct3/4 Sox2, CKG Oct3/4 Sox2, PCMLK Oct3/4 Sox2, PMLK Oct3/4 Sox2, CMLK Oct3/4 Sox2, PCMLG Oct3/4 Sox2, PMLG Oct3/4 Sox2, CMLG Oct3/4 Sox2, PCMKG Oct3/4 Sox2, PMKG Oct3/4 Sox2, CMKG Oct3/4 Sox2, PCLKG Oct3/4 Sox2, PLKG Oct3/4 Sox2, CLKG Oct3/4 Sox2, PCMLKG Oct3/4 Sox2, PMLKG Oct3/4 Sox2, CMLKG Oct3/4 Sox2, PCM Lin-28 Sox2, PM Lin-28 Sox2, CM Lin-28 Sox2, PCL Lin-28 Sox2, PL Lin-28 Sox2, CL Lin-28 Sox2, PCK Lin-28 Sox2, PK Lin-28 Sox2, CK Lin-28 Sox2, PCG Lin-28 Sox2, PG Lin-28 Sox2, CG Lin-28 Sox2, PCML Lin-28 Sox2, PML Lin-28 Sox2, CML Lin-28 Sox2, PCMK Lin-28 Sox2, PMK Lin-28 Sox2, CMK Lin-28 Sox2, PCMG Lin-28 Sox2, PMG Lin-28 Sox2, CMG Lin-28 Sox2, PCLK Lin-28 Sox2, PLK Lin-28 Sox2, CLK Lin-28 Sox2, PCLG Lin-28 Sox2, PLG Lin-28 Sox2, CLG Lin-28 Sox2, PCKG Lin-28 Sox2, PKG Lin-28 Sox2, CKG Lin-28 Sox2, PCMLK Lin-28 Sox2, PMLK Lin-28 Sox2, CMLK Lin-28 Sox2, PCMLG Lin-28 Sox2, PMLG Lin-28 Sox2, CMLG Lin-28 Sox2, PCMKG Lin-28 Sox2, PMKG Lin-28 Sox2, CMKG Lin-28 Sox2, PCLKG Lin-28 Sox2, PLKG Lin-28 Sox2, CLKG Lin-28 Sox2, PCMLKG Lin-28 Sox2, PMLKG Lin-28 Sox2, CMLKG Lin-28 Sox2, PCM Lin-28 Oct3/4 Sox2, PM Lin-28 Oct3/4 Sox2, CM Lin-28 Oct3/4 Sox2, PCL Lin-28 Oct3/4 Sox2, PL Lin-28 Oct3/4 Sox2, CL Lin-28 Oct3/4 Sox2, PCK Lin-28 Oct3/4 Sox2, PK Lin-28 Oct3/4 Sox2, CK Lin-28 Oct3/4 Sox2, PCG Lin-28 Oct3/4 Sox2, PG Lin-28 Oct3/4 Sox2, CG Lin-28 Oct3/4 Sox2, PCML Lin-28 Oct3/4 Sox2, PML Lin-28 Oct3/4 Sox2, CML Lin-28 Oct3/4 Sox2, PCMK Lin-28 Oct3/4 Sox2, PMK Lin-28 Oct3/4 Sox2, CMK Lin-28 Oct3/4 Sox2, PCMG Lin-28 Oct3/4 Sox2, PMG Lin-28 Oct3/4 Sox2, CMG Lin-28 Oct3/4 Sox2, PCLK Lin-28 Oct3/4 Sox2, PLK Lin-28 Oct3/4 Sox2, CLK Lin-28 Oct3/4 Sox2, PCLG Lin-28 Oct3/4 Sox2, PLG Lin-28 Oct3/4 Sox2, CLG Lin-28 Oct3/4 Sox2, PCKG Lin-28 Oct3/4 Sox2, PKG Lin-28 Oct3/4 Sox2, CKG Lin-28 Oct3/4 Sox2, PCMLK Lin-28 Oct3/4 Sox2, PMLK Lin-28 Oct3/4 Sox2, CMLK Lin-28 Oct3/4 Sox2, PCMLG Lin-28 Oct3/4 Sox2, PMLG Lin-28 Oct3/4 Sox2, CMLG Lin-28 Oct3/4 Sox2, PCMKG Lin-28 Oct3/4 Sox2, PMKG Lin-28 Oct3/4 Sox2, CMKG Lin-28 Oct3/4 Sox2, PCLKG Lin-28 Oct3/4 Sox2, PLKG Lin-28 Oct3/4 Sox2, CLKG Lin-28 Oct3/4 Sox2, PCMLKG Lin-28 Oct3/4 Sox2, PMLKG Lin-28 Oct3/4 Sox2, CMLKG Lin-28 Oct3/4 Sox2, PC LIN-28, P LIN-28, C LIN-28, PC OCT3/4, P OCT3/4, C OCT3/4, PC SOX2, P SOX2, C SOX2, PC LIN-28 OCT3/4, P LIN-28 OCT3/4, C LIN-28 OCT3/4, PC LIN-28 SOX2, P LIN-28 SOX2, C LIN-28 SOX2, PC OCT3/4 SOX2, P OCT3/4 SOX2, C OCT3/4SOX2, PC LIN-28 OCT3/4 SOX2, P LIN-28 OCT3/4 SOX2, and C LIN-28 OCT3/4 SOX2, wherein P represents PRDM16, C represents C/EBPβ, M represents c-Myc, L represents L-Myc, K represents KLF-4, and G represents Glis1. Among these, for example, PCM, CM, PCL, CL, PCG, CG, PCML, CML, PCM Oct3/4, CM Oct3/4, PCMG, CMG, PCL Oct3/4, CL Oct3/4, PCLG, CLG, PCML Oct3/4, CML Oct3/4, PCMLG, CMLG, PCM Oct3/4 G, CM Oct3/4 G, PCL Oct3/4 G, CL Oct3/4 G, PCML Oct3/4 G, and CML Oct3/4 G are preferable. Among these, PCM, CM, PCL, CL, PCML, CML, PCM Oct3/4, CM Oct3/4, PCMG, CMG, PCL Oct3/4, CL Oct3/4, PCLG, CLG, PCML Oct3/4, CML Oct3/4, PCMLG, CMLG, PCM Oct3/4 G, CM Oct3/4 G, PCL Oct3/4 G, CL Oct3/4 G, PCML Oct3/4 G, and CML Oct3/4 G are particularly preferable. Among these, PCM, CM, PCML, CML, PCMG, CMG, PCLG, CLG, PCMLG, and CMLG are further preferable.

c-Myc can be replaced by another Myc family gene (N-Myc, L-Myc, S-Myc, and B-Myc). In the present specification, the present invention is described using "c-Myc" and "L-Myc" as typical Myc family genes. However, other Myc family genes can also be used in the same manner as c-Myc.

KLF-4 can be replaced by another Klf family gene (KLF1, KLF2, KLF3, KLF5, KLF6, KLF7, KLF8, KLF9, KLF10, KLF11, KLF12, KLF13, KLF14, KLF15, KLF16, and KLF17). In the present specification, the present invention is described using "KLF-4" as a typical Klf family gene. However, other Klf family genes can also be used in the same manner as KLF-4.

GLIS1 (GLIS family zinc finger 1) can be replaced by another GLIS family member gene, such as GLIS 2 or GLIS 3.

Likewise, Oct3/4 can be replaced by another Oct family gene, and Sox2 can be replaced by another Sox family gene.

All of the above genes are highly conserved among vertebrates. The term "gene" referred to in the present specification includes its homologues, unless the name of a particular animal is indicated. "Gene" also encompasses polymorphisms and mutated genes that have a function comparable to that of wild-type gene products. Genes to be introduced are preferably genes derived from the same mammal as that of the somatic cells. For example, human genes are introduced into human somatic cells.

The method of the present invention can be performed according to a known direct reprogramming method, except that specific genes are selected. The method of the present invention can be performed, for example, according to the methods of documents 1 to 6 below:
Document 1 - Direct reprogramming of fibroblasts into functional cardiomyocytes by defined factors. Masaki Ieda, Ji-Dong Fu, Paul Delgado-Olguin, Vasanth Vedantham, Yohei Hayashi, Benoit G. Bruneau, and Deepak Srivastava. Cell 142: 375-386, 2010
Document 2 - Direct conversion of fibroblasts to functional neurons by defined factors. Thomas Vierbuchen, Austin Ostermeier, Zhiping P. Pang, Yuko Kokubu, Thomas C. Sudhof & Marius Wernig. Nature 463: 1035-1041, 2010
Document 3 - Induction of human neuronal cells by defined transcription factors. Pang ZP, Yang N, Vierbuchen T, Ostermeier A, Fuentes DR, Yang TQ, Citri A, Sebastiano V, Marro S, Sudhof TC, Wernig M. Nature 476: 220-223, 2011
Document 4 - Generation of hyaline cartilaginous tissue from mouse adult dermal fibroblast culture by defined factors. Kunihiko Hiramatsu, Satoru Sasagawa, Hidetatsu Outani, Kanako Nakagawa, Hideki Yoshikawa, and Noriyuki Tsumaki, Journal of Clinical Investigation. 121: 640-657, 2011
Document 5 - Induction of functional hepatocyte-like cells from mouse fibroblasts by defined factors. Pengyu Huang, Zhiying He, Shuyi Ji, Huawang Sun, Dao Xiang, Changcheng Liu, Yiping Hu, XinWang & Lijian Hui. Nature 475: 386-389, 2011
Document 6 - Direct conversion of mouse fibroblasts to hepatocyte-like cells by defined factors. Sayaka Sekiya & Atsushi Suzuki. Nature 475: 390-393, 2011.

More specifically, it is preferable that the transgenes for conversion to brown adipocytes are incorporated into expression vectors, the expression vectors are introduced into target somatic cells and the genes are intracellularly expressed.

Examples of methods for introducing genes include a method of infection with a viral vector, such as a retroviral vector, an adenoviral vector, a lentiviral vector, an adeno-associated viral vector, a herpes viral vector, or a Sendai viral vector. When a gene and expression product thereof is introduced, a method of transfection of a plasmid vector, an episomal vector, or the expression product of the gene (RNA, protein) using a non-viral vector, such as a cationic liposome, a cationic polymer, or electroporation is also usable. RNA can also be introduced. All the above gene transfer means are collectively referred to herein as vectors.

When a drug selection marker gene (conferring resistance to puromycin, blasticidin S, neomycin, hygromycin, etc.) is introduced with therapeutic genes and then drug selection is performed, cells expressing the genes necessary for conversion to brown adipocytes can be selected and used.

When an introduced factor is an expression product of a gene (for example, protein), the factor may be introduced into somatic cells by binding a peptide called a protein transduction domain (PTD) to the expression product protein, and adding the fusionprotein to a culture medium. If some of the genes necessary for conversion to brown adipocytes have been expressed in somatic cells for use as the starting material for brown adipocytes, it is not necessary to introduce the proteins externally.

The differentiation-inducing medium for induction into brown adipocytes is not particularly limited, and ordinary cell culture medium may be used.

The differentiation-inducing medium for induction into brown adipocytes is not particularly limited, and ordinary cell culture medium may be used. For example, although the medium is not limited thereto, the following known brown fat induction medium type I and brown fat induction medium type II may be used: brown fat induction medium type I (medium obtained by adding 850 nM human insulin, 1 nM triiodothyronine (T3), 0.5 mM 3-isobutyl-1-methylxanthine (IBMX), 100 nM dexamethazone, 125 nM indometacin, and 1 µg/ml rosiglitazone (all of these concentrations are final concentrations) to 1% NEAA 10% FBS DMEM containing 100 U/mL penicillin and 100 µg/ml streptomycin); and brown fat induction medium type II (medium obtained by adding 850 nM human insulin, 1 nM triiodothyronine (T3), 1 µg/mL rosiglitazone (all of these concentrations are final concentrations) to 1% NEAA 10% FBS DMEM containing 100 U/mL penicillin and 100 µg/ml streptomycin).

The obtainment of brown adipocytes can be evaluated by measuring the expression of a gene such as UCP1, CIDEA, PGC1, DIO2, Cox8b, or Otop. It can also be evaluated by global gene expression profiling analysis.

In order to prevent immune response after transplantation, the cells to be transplanted for prevention or treatment are preferably autologous cells established from the patient.

The gene transfer of the present invention may be performed using a plasmid. Viral vectors, for example, retroviral vectors, may also be used. Viral vectors are preferable in view of transfer efficiency and stable maintenance of transgenes, and plasmids are preferable in view of suppression of the risk of oncogenesis.

The genes to be introduced into somatic cells can be transcribed by an LTR promoter, or may be expressed from another promoter inside vectors. For example, a constitutive expression promoter such as a CMV promoter, EF-1α promoter, or CAG promoter, or a desired inducible promoter may be used. Alternatively, a chimeric promoter, in which a portion of LTR is substituted with another promoter, may be used.

Examples of diseases to be treated using brown adipocytes (grafting material) obtained by the present invention include obesity, visceral fat obesity, adiposis, diabetes, type I diabetes, type II diabetes, diabetic retinopathy, diabetic neuropathy, diabetic nephropathy, delayed wound healing, impaired glucose tolerance, insulin resistance, hyperglycemia, hyperinsulinemia, cataract, glaucoma, retinopathy, neuropathy, nephropathy, periodontal disease, skin diseases, gangrene, ulcer, abnormal lipid metabolism, hyperfattyacidemia , hypertriglyceridemia, hyperglycerolemia, hypercholesterolemia, hyperlipidemia, hypo-high-density-lipoproteinemia, Syndrome X, microangiopathy, arteriosclerotic disease, arteriosclerosis obliterans, cerebrovascular disease, coronary artery disease, atherosclerosis, arteriosclerosis, aneurysm, hyperglycemia (in particular, postprandial hyperglycemia), hypertension, hyperuricemia, gout, chronic systemic inflammation, non-alcoholic fatty liver disease, fatty liver, liver cirrhosis, hepatogenous diabetes, cholecystitis, gallstone, urinary incontinence, urinary retention, impotence, cystitis, endothelial dysfunction, autonomic disorders, eczema, stomatitis, pyorrhea alveolaris, osteoporosis, metabolic syndrome, and the like.

Unless otherwise indicated, the term "treatment" as used herein means any treatment that is applied to a patient while the patient is suffering from a specific disease or disorder and that can reduce the severity of the disease or disorder or one or more symptoms thereof, or retard or slow the progression of the disease or disorder. The term "treatment" as used herein includes "prevention."

Brown adipocytes obtained by the present invention can be used not only for the treatment of disease, but also in beauty applications. For example, to reduce adipose tissue for weight loss, brown adipocytes may be transplanted. Any treatment provided to humans in beauty applications is also called treatment for reasons of convenience in the present specification. In this case, reference to a "patient" can be deemed to refer to a "healthy person" or a "human," and reference to "disease" can be deemed to refer to "beauty."

The present invention can also be used in the treatment of disease not only for humans, but also for mammals including animals kept as pets, such as dogs and cats, and animals kept as livestock, such as cattle, horses, pigs, sheep, and chickens. In this case, reference to a "patient" can be deemed to refer to a "diseased animal" or "mammal."

The term "grafting material" refers to a material, comprising brown adipocytes, to be introduced into a living body for the treatment of obesity, diabetes, impaired glucose tolerance, abnormal lipid metabolism, or metabolic syndrome, or for beauty treatment. "Grafting material" encompasses a material that is to be transplanted into the same or different individuals after tissue construction is formed *in vitro.* Brown adipocytes obtained by the present invention may be used for preparing a grafting material. The brown adipocytes themselves can also be used as a grafting material. Accordingly, the brown adipocytes may be transplanted into a patient or a subject as a cell preparation, or transplanted together with a substrate (scaffold) made of artificial material.

The somatic cells are preferably derived from a mammal. When brown adipocytes are transplanted into a living body, it is preferable to use somatic cells derived from the subject into which the brown adipocytes are to be transplanted (autologous cells), in view of reducing risks such as infection and graft rejection. However, not only autologous cells, but also brown adipocytes prepared beforehand from the somatic cells of another person or another animal may be used for transplantation. Alternatively, brown adipocytes may be obtained from somatic cells prepared beforehand of another person or another animal, and used for transplantation. More specifically, a bank of brown adipocytes or a bank of brown adipocyte precursor cells may be prepared and used for transplantation. In such a case, MHC typing may be performed beforehand to reduce risks such as graft rejection. The character, tumorigenicity, etc., of brown adipocytes may also be confirmed beforehand.

In the present specification, examples of mammals include mice, rats, hamsters, humans, dogs, cats, monkeys, rabbits, cattle, horses, pigs, and the like, in particular humans.

The present invention can also be used for various research, technical developments, etc., that use brown adipocytes. For example, the present invention is useful in basic research such as analysis of the development and aging of brown adipocytes and the metabolic regulation mechanism of brown adipocytes, as well as analysis of the influences of nutrition, immunity, nerves, hormones, and food thereon.

With the use of the present invention, brown adipocytes can be established from humans or animals with various diseases or genetic backgrounds simply, rapidly, and inexpensively. Therefore, brown adipocyte abnormalities associated with diseases or genetic backgrounds can be analyzed by a biochemical, molecular biological, immunological, or like method. Such analysis can aid in research, for instance, elucidation of pathogenesis of various diseases including obesity, diabetes, impaired glucose tolerance, abnormal lipid metabolism, and metabolic syndrome, and aid in the development of diagnostic methods. For example, the present invention can be applied to personalized medicine by determining or predicting, in such diseases, differences resulting from genetic differences, such as difference in incidence, difference in the degree of disease progression, difference in response to treatment, and difference in therapeutic effects. In addition, developing drugs, performing drug toxicity tests, or the like, using such brown adipocytes can contribute to the development of new therapies for various diseases, for example, obesity, diabetes, impaired glucose tolerance, abnormal lipid metabolism, and metabolic syndrome.

Another gene, or other genes, can be further added to the combination of genes of the present invention.

The present invention can provide brown adipocytes from somatic cells in a short period of time by direct reprogramming or a method analogous thereto. Since these brown adipocytes can be easily induced from somatic cells of a subject into which the brown adipocytes are to be transplanted, immunological rejection or like problems do not arise in transplantation. Further, brown adipocytes can be directly induced from somatic cells without passing through iPS cells or ES cells, which avoids problems attributed to pluripotent stem cells, such as oncogenesis.

### Examples

Examples are shown below; however, the present invention is not limited to these Examples.

### Example 1

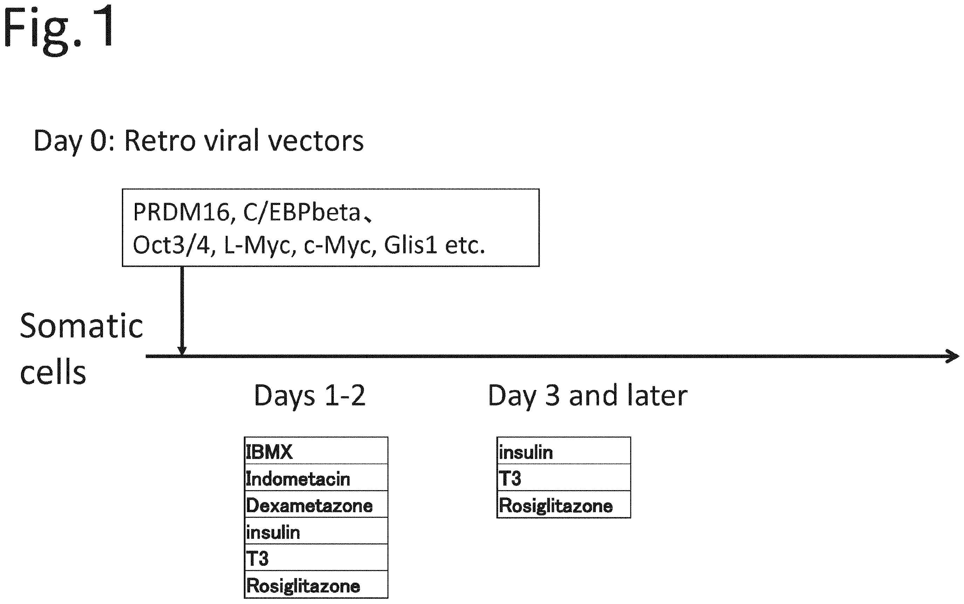

### Scheme of some experiments (Fig. 1)

The cDNA coding sequences of various genes such as C/EBPβ were incorporated into pMXs-puro retroviral vector plasmids using a Gene art system. Plat GP packaging cells were suspended in 1% NEAA 10% FBS DMEM (ordinary medium) containing 100 U/mL penicillin and 100 µg/ml streptomycin, and plated in a gelatin-coated 10 cm culture dish at a concentration of 5 × 10⁶ cells/dish (day -3). After culturing for 24 hours, the pMXs vectors containing various genes were introduced in various combinations in the following proportion together with pCMV VSV vectors, using X-tremeGENE 9. More specifically, a mixture of 5 µg transgenes, 2.5 µg pCMV-VSV, 500 µl Opti-MEM, and 22.5 µl X-tremeGENE 9 was added to 10 cm dish containing 10 ml of medium (day -2). After 24 hours, the medium was replaced with antibiotic-free ordinary medium (day -1). On the same day (day - 1), normal human dermal fibroblast line aHDFs or human adipose-derived stem cells ADSCs were plated on culture dishes or 12-well plates at 1.5 × 10⁴ to 2 × 10⁴ cells/mL. After 24 hours (day 0), the Plat GP culture supernatant was passed through a syringe filter with a pore diameter of 0.45 µm, and then mixed with polybrene (final concentration of 4 µg/mL) (virus suspension). The culture supernatant of aHDFs was removed by suction, and then 1 mL of the virus suspension was quickly added, followed by culturing for 24 hours (infection). Non-virus-infected cells were prepared as a control group. One day later (day 1), the culture supernatant was removed by suction, and brown fat induction medium type I (medium obtained by adding 850 nM human insulin, 1 nM triiodothyronine (T3), 0.5 mM 3-isobutyl-1-methylxanthine (IBMX), 100 nM dexamethazone, 125 nM indometacin, and 1 µg/ml rosiglitazone (all of these concentrations are final concentrations) to ordinary medium) was added, followed by culturing for 2 days. On day 3, the medium was removed by suction, and brown fat induction medium type II (medium obtained by adding 850 nM human insulin, 1 nM triiodothyronine (T3), and 1 µg/mL rosiglitazone (all of these concentrations are final concentrations) to ordinary medium) was added. Thereafter, the medium was replaced every two days with fresh culture medium having the same composition. On day 12 to day 22, Oil Red O staining and real-time RT-PCR were performed. Cells that were cultured in the same manner but not infected with retroviral vectors were used as a control.

### Example 2

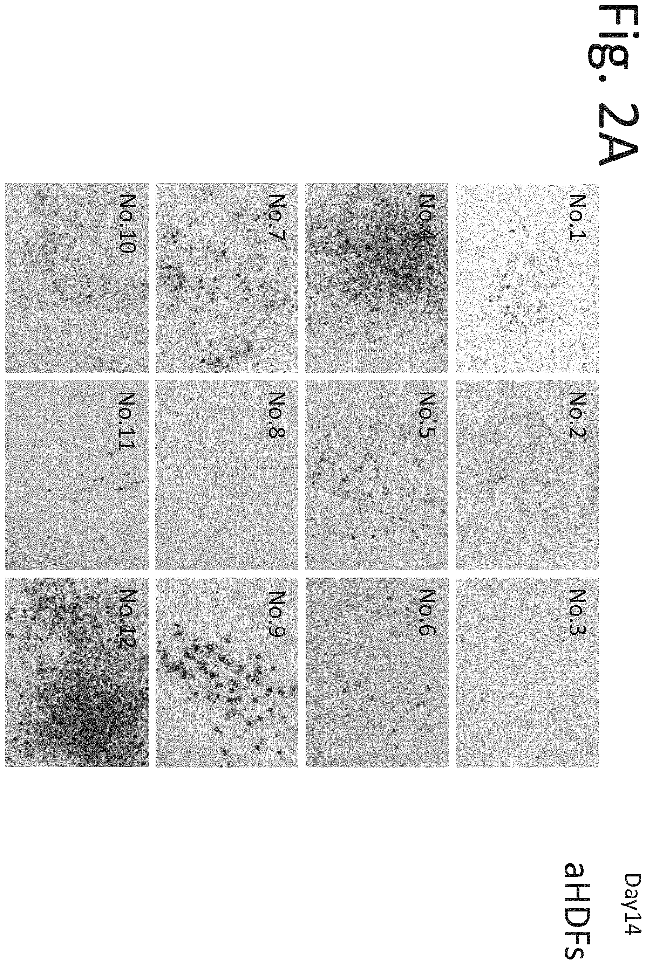

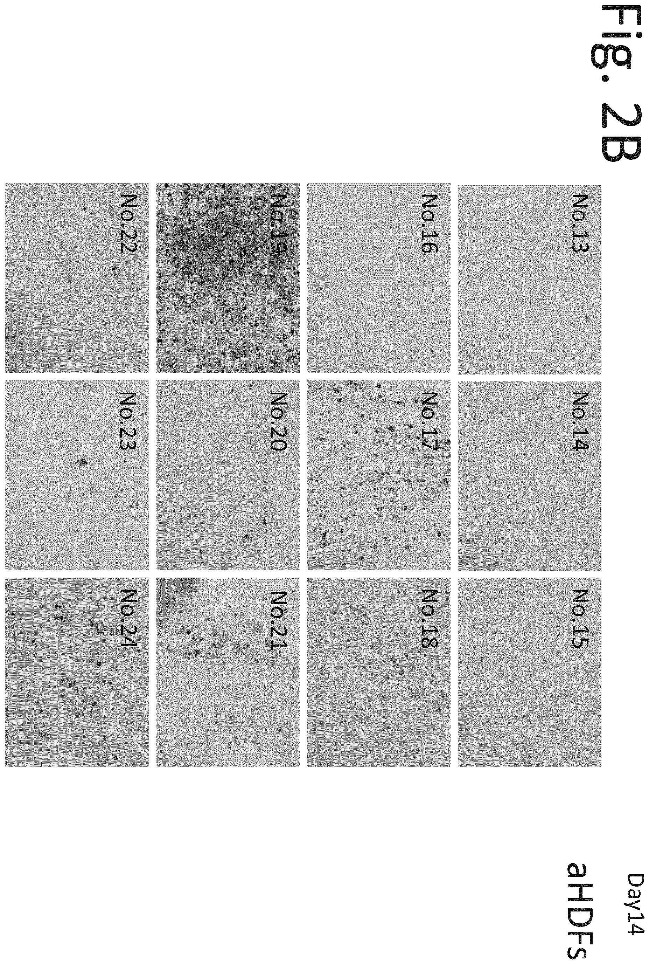

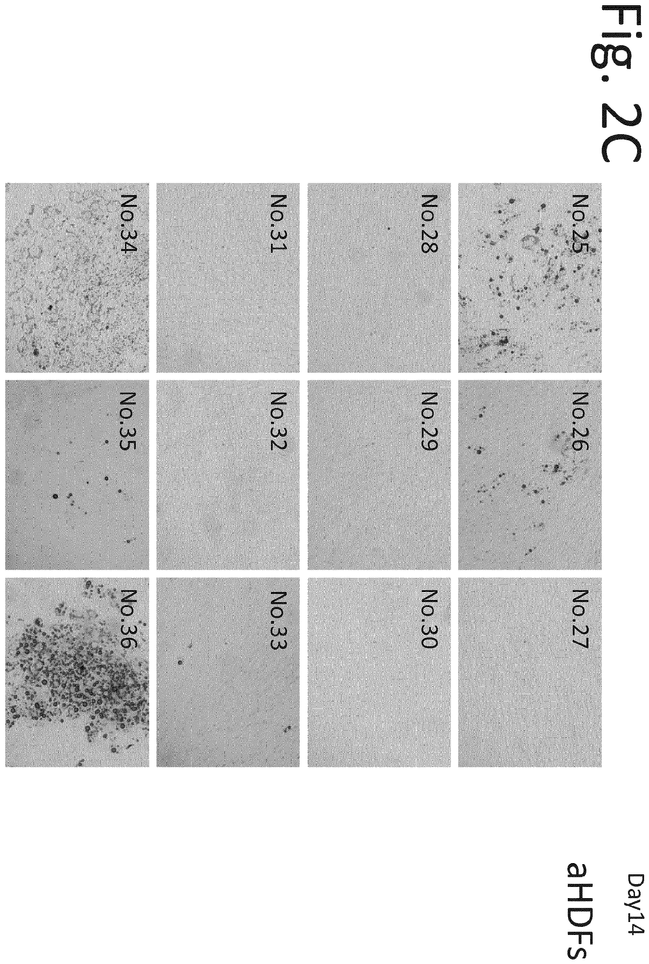

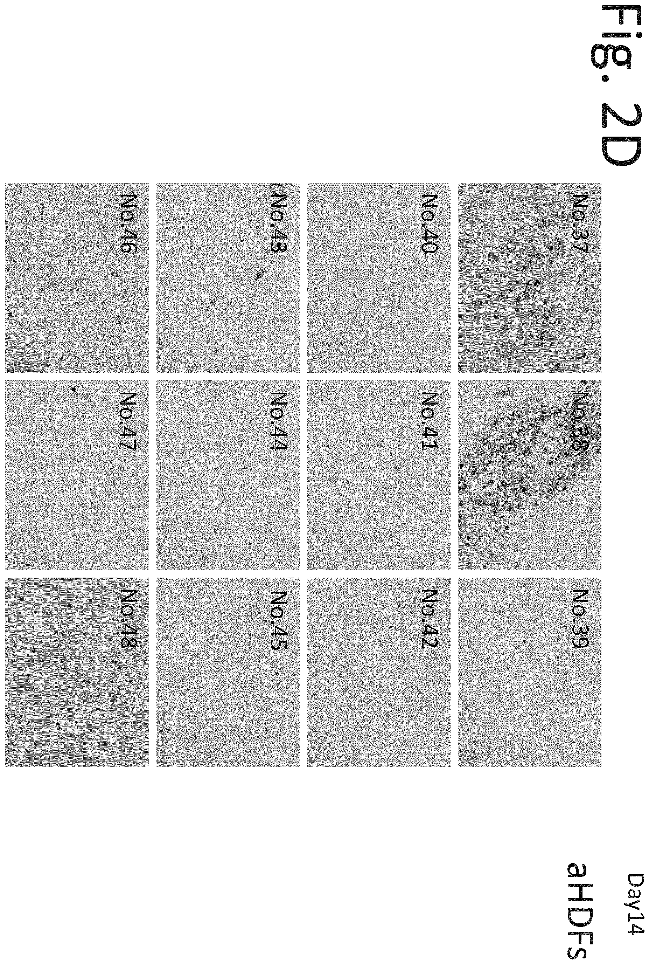

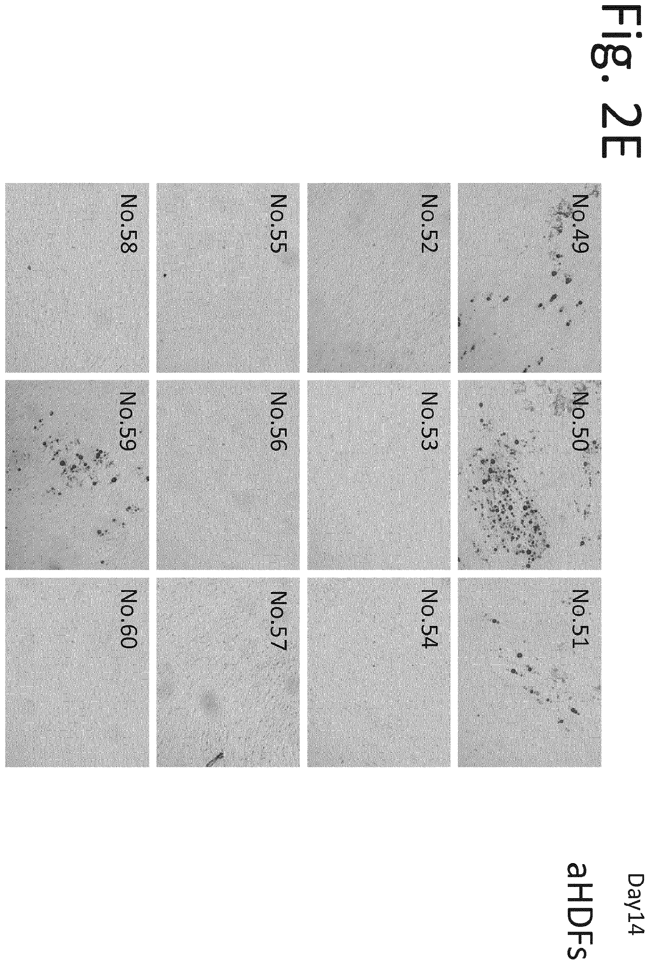

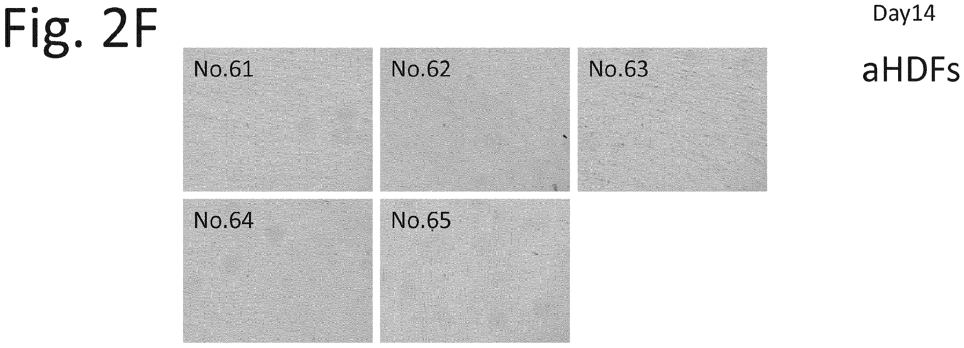

### Conversion from normal human dermal fibroblasts into brown adipocytes, images of Oil Red O staining (Fig. 2)

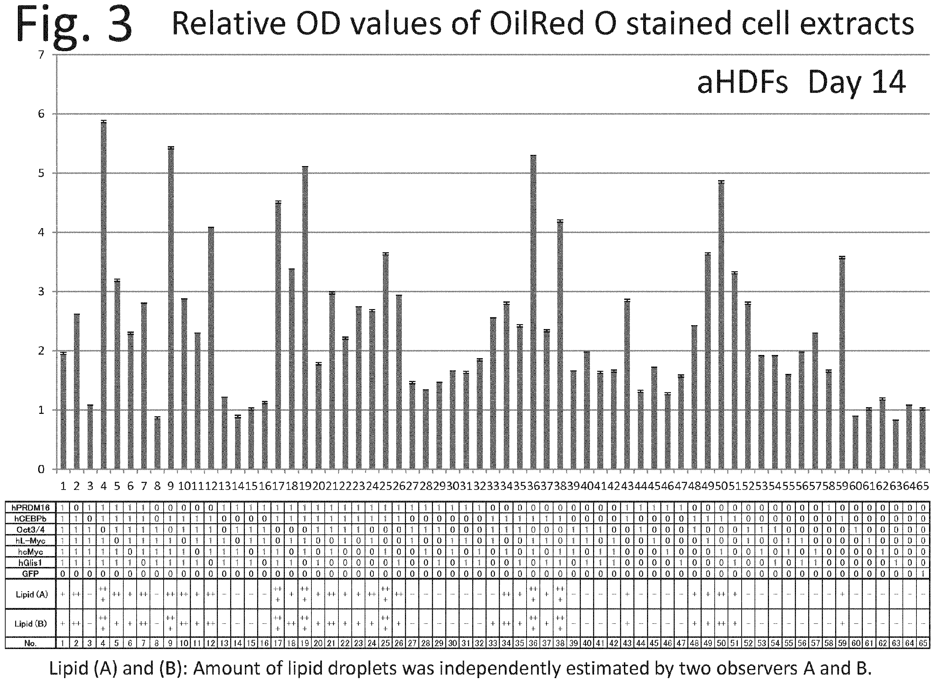

Normal human dermal fibroblasts aHDFs were cultured in 12-well plates, and an experiment was performed as shown in Fig. 1. On day 14, the culture medium was removed by suction from each well, and the cells were washed with PBS once and then fixed with 60% isopropanol. An Oil Red O staining solution was added, and the plates were allowed to stand at 37°C for 15 minutes (the Oil Red O staining solution was prepared as follows: 0.24 g of an Oil Red O powder was dissolved in 30 mL of 99.7% isopropanol, 20 mL of distilled water was added thereto, the resulting mixture was allowed to stand at room temperature for 30 minutes and filtered using a filter paper, and the obtained solution was used as an Oil Red O staining solution). The cells were washed with 60% isopropanol once, and then washed with pure water three times. Figs. 2A-F show the images of the plates. In each well of the plates, a different gene combination was introduced. Fig. 3 shows which gene combination was introduced in which number well (in the table shown at the bottom of Fig. 3, "No." refers to the same No. as in Fig. 2 and indicate well numbers in the plates. In the column of each gene, "1" means that the cells were infected with a retroviral vector containing the gene, whereas a blank means that the cells were not infected with a retroviral vector containing the gene). Those stained red indicate lipid. In some wells, brown adipocytes containing small multilocular lipid droplets were observed. For example, well No. 36 in Fig. 2, which contained cells infected with retroviral vectors containing C/EBPβ, L-Myc, and c-Myc genes as shown in No. 36 of Fig. 3, was shown to contain numerous brown adipocytes.

### Example 3

### Conversion from normal human dermal fibroblasts into brown adipocytes, semi-quantitation and quantitation of Oil Red O staining (Fig. 3)

To semi-quantify the Oil Red O staining in the same experiment as that in Fig. 2, two evaluators independently observed the plates with a stereoscopic microscope to evaluate the Oil Red O staining on a 4-point scale (+++, ++, +, - in descending order of Oil Red O staining strength). The table at the bottom of Fig. 3 shows the results. In the column of each gene in the table at the bottom of Fig. 3, "1" means that the cells were infected with a retroviral vector containing the gene, whereas a blank means that the cells were not infected with a retroviral vector containing the gene.

To quantify lipid content in the same experiment as that in Fig. 2, the distilled water was removed from each well after taking images, and 300 µl of 100% isopropanol was added to prepare extracts. 250 µl of each extract was transferred to a 96-well plate. The absorbance at a wavelength of 550 nm (OD550) was then measured with a microplate reader. Fig. 3 shows the results. The vertical axis of the graph indicates OD550, showing lipid content in each well. For example, the cells infected with retroviral vectors containing PRDM16, C/EBPβ, L-Myc, c-Myc, and Glis1 genes in No. 4 were shown to contain the largest number of brown adipocytes.

### Example 4

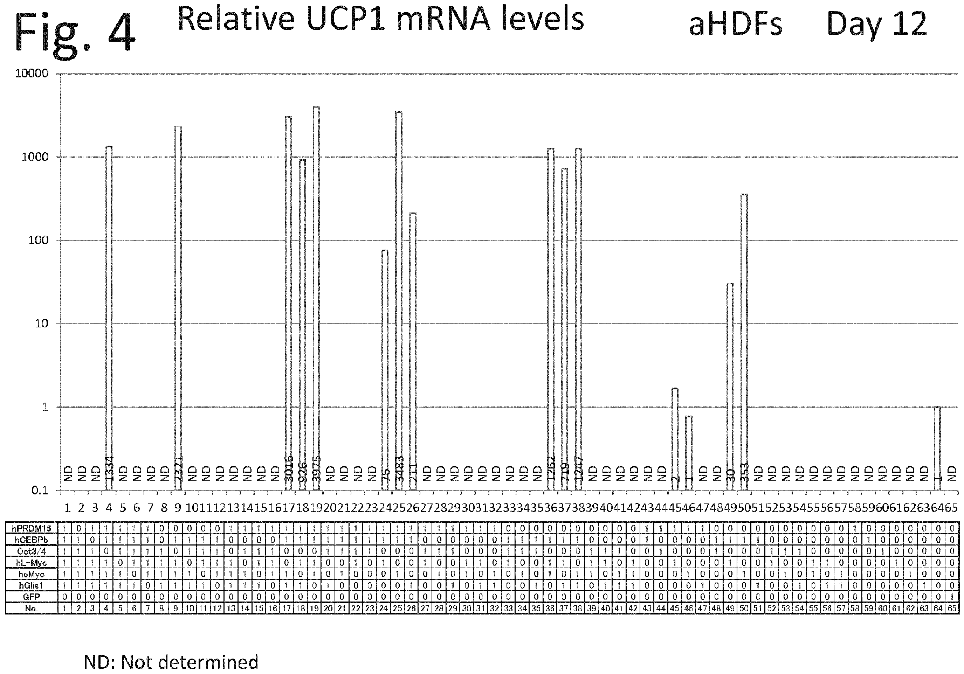

### Conversion from normal human dermal fibroblasts into brown adipocytes, measurement of mRNA expression of UCP1 gene (Fig. 4)

Normal human dermal fibroblasts aHDFs were cultured in 12-well plates, and an experiment was performed as shown in Fig. 1. Twelve days after gene transfer, total RNA was collected from some wells using ISOGEN II, and cDNA was synthesized using ReverTra Ace qPCR RT Master Mix. Real-time PCR Master Mix, a TaqMan probe, specific primers, and cDNA were mixed, and real-time RT-PCR was performed using an AB7300 Real-Time PCR System to quantify the mRNA levels of UCP1 and β-actin genes. The value of the UCP1 mRNA level with respect to the β-actin mRNA level of the cells in each well was calculated. Fig. 4 shows the results. In the column of each gene in the table at the bottom of Fig. 4, "1" means that the cells were infected with a retroviral vector containing the gene, whereas a blank means that the cells were not infected with a retroviral vector containing the gene. The vertical axis of the graph indicates relative values that were calculated on the assumption that the value in No. 64 (cells in which no genes were introduced) was 1; the numbers inside the bars indicate the same. ND (Not determined) means that measurement was not performed in this experiment. The strongest expression, compared to the control, of uncoupling protein-1 (UCP-1), which is a brown adipocyte-specific marker at the gene level, was observed in the cells in which the four genes PRDM16, C/EBPβ, L-Myc, and c-Myc were introduced (No. 19). In addition, an extremely high expression of UCP1 was also observed in the cells in which the three genes C/EBPβ, L-Myc, and c-Myc were introduced (No. 36), and in the cells in which the three genes C/EBPβ, c-Myc, and Glis1 were introduced (No. 38). These results show that direct reprogramming of human fibroblasts into brown adipocytes can be efficiently performed with these genes, even when PRDM16 is not used.

### Example 5

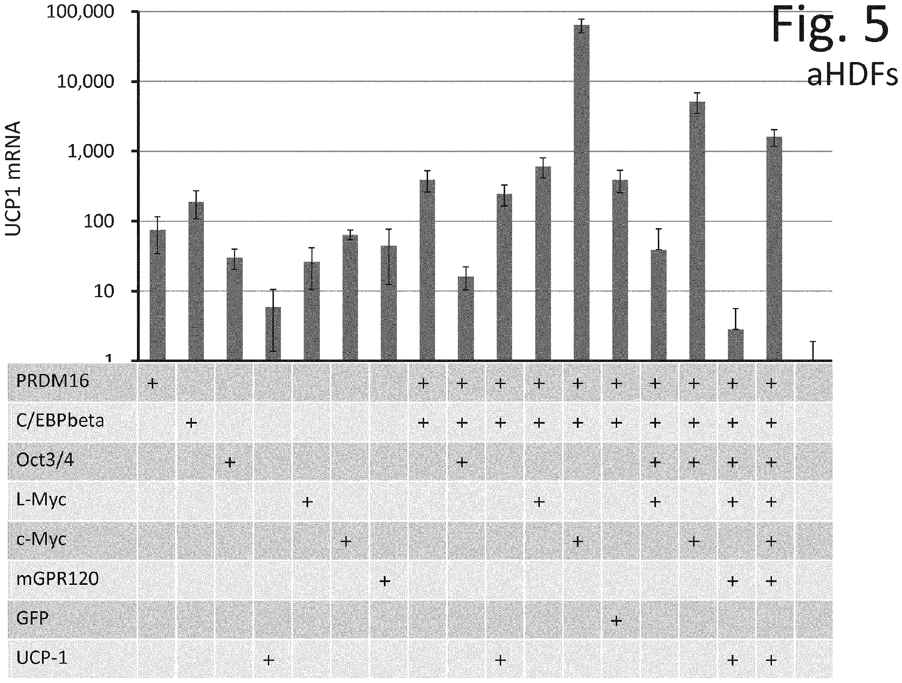

### Conversion from normal human dermal fibroblasts into brown adipocytes, the mRNA expression of the UCP1 gene (Fig. 5)

Normal human dermal fibroblasts aHDFs were cultured in 12-well plates, and an experiment was performed as shown in Fig. 1 (this experiment was carried out independently with combinations of genes that were different from those shown in Fig. 4). Twelve days after gene transfer, total RNA was collected from some wells using ISOGEN II, and cDNA was synthesized using ReverTra Ace qPCR RT Master Mix. Real-time PCR Master Mix, a TaqMan probe, specific primers, and cDNA were mixed, and real-time RT-PCR was performed using a AB7300 Real-Time PCR System to quantify the mRNA levels of UCP1 and β-actin genes. The value of the UCP1 mRNA level with respect to the β-actin mRNA level of the cells in each well was calculated. Fig. 5 shows the results. In the column of each gene in the table at the bottom of Fig. 5, "+" means that the cells were infected with a retroviral vector containing the gene, whereas a blank means that the cells were not infected with a retroviral vector containing the gene. The vertical axis of the graph indicates relative values that were calculated on the assumption that the value in the cells into which no genes were introduced (rightmost bar) was 1. The strongest expression of uncoupling protein-1 (UCP-1), which is a brown adipocyte-specific marker, was observed at the mRNA level in the cells in which the three genes PRDM16, C/EBPβ, and c-Myc were introduced (the 7th bar from the right). The expression level was shown to be 100 times or more higher than the mRNA level of UCP1 observed in the cells in which the two genes PRDM16 and C/EBPβ were introduced (the 8th bar from the left).

### Example 6

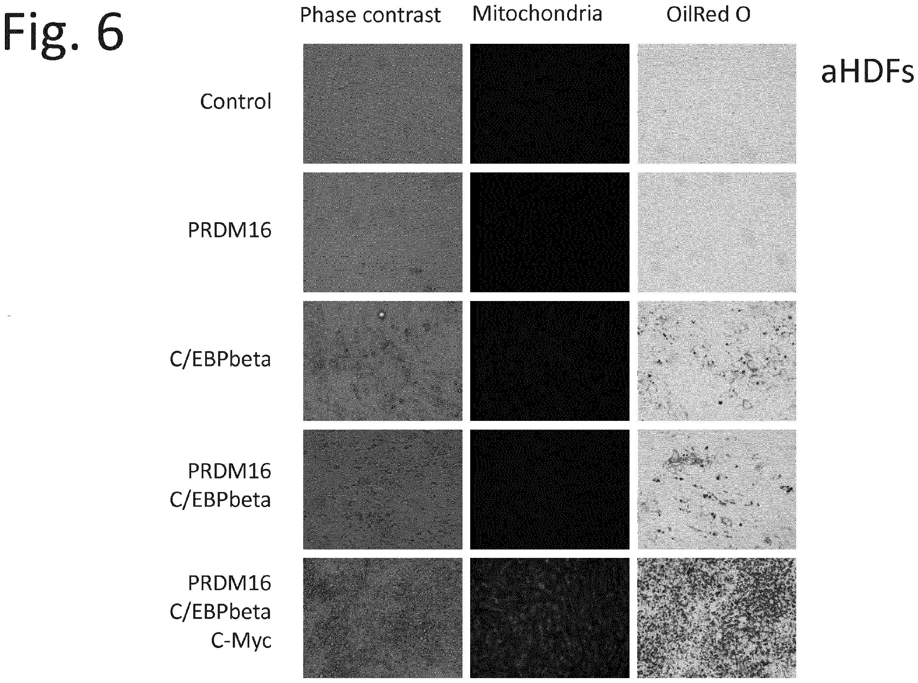

### Conversion from normal human dermal fibroblasts into brown adipocytes, images of mitochondrial staining (Fig. 6)

Normal human dermal fibroblasts aHDFs were cultured in 12-well plates, and an experiment was performed as shown in Fig. 1. Twelve days after gene transfer, the cells were observed with a phase contrast microscope (left column in Fig. 6). To visualize mitochondria, Mito Tracker Red probe produced by Invitrogen was added to the culture medium in some wells to a final concentration of 200 nM. The cells were allowed to stand in 5% CO₂/95% air at 37°C for 15 minutes, and then observed with a fluorescence microscope produced by Olympus Corporation (middle column in Fig. 6). The cells in some other wells were stained with Oil Red O in the same manner as in Fig. 2 (right column in Fig. 6). In the group in which the three genes PRDM16, C/EBPβ, and c-Myc were introduced, numerous cells with numerous mitochondria accumulated therein were observed. In addition, the Oil Red O staining confirmed brown adipocytes containing small multilocular lipid droplets in this group. Compared to this group, the amounts of mitochondria and lipid droplets were both far lower in the group in which the two genes PRDM16 and C/EBPβ were introduced.

### Example 7

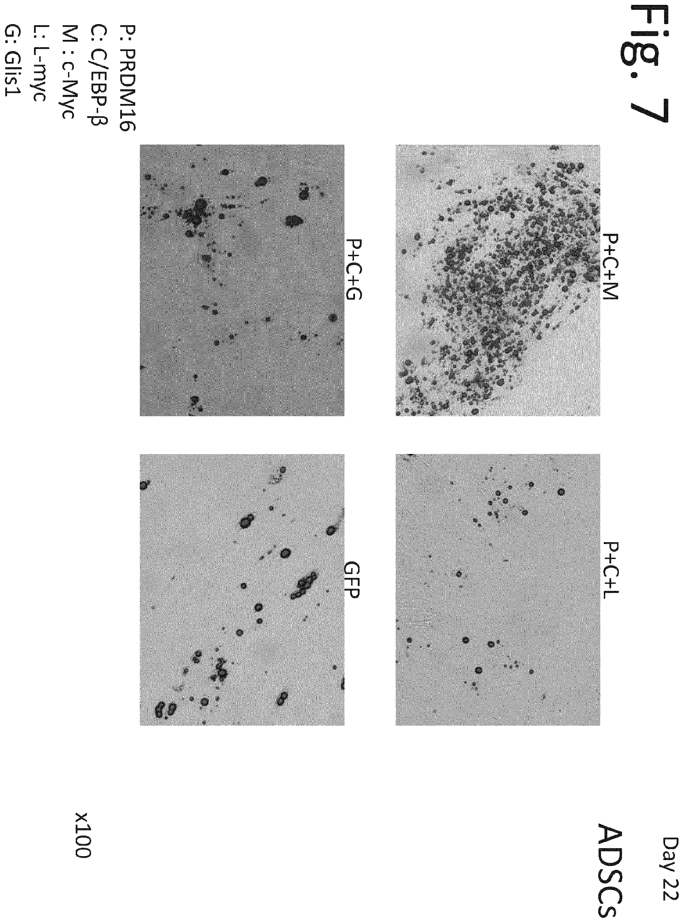

### Conversion from human adipose-derived stem cells into brown adipocytes, images of Oil Red O staining (Fig. 7)

Human adipose-derived stem cells (ADSCs) were cultured in 12-well plates, and an experiment was performed as shown in Fig. 1. Twenty-two days after gene transfer, the cells were stained with Oil Red O in the same manner as in Fig. 2. Fig. 7 shows the results. Those stained red indicate lipid, showing induction into adipocytes. Numerous brown adipocytes containing small multilocular lipid droplets were observed in the cells infected with retroviral vectors containing PRDM16, C/EBPβ, and c-Myc genes (upper left in Fig. 7). In contrast, white adipocytes containing large unilocular lipid droplets were observed in the cells in which the green fluorescent protein (GFP) gene was introduced (lower right in Fig. 7).

### Example 8

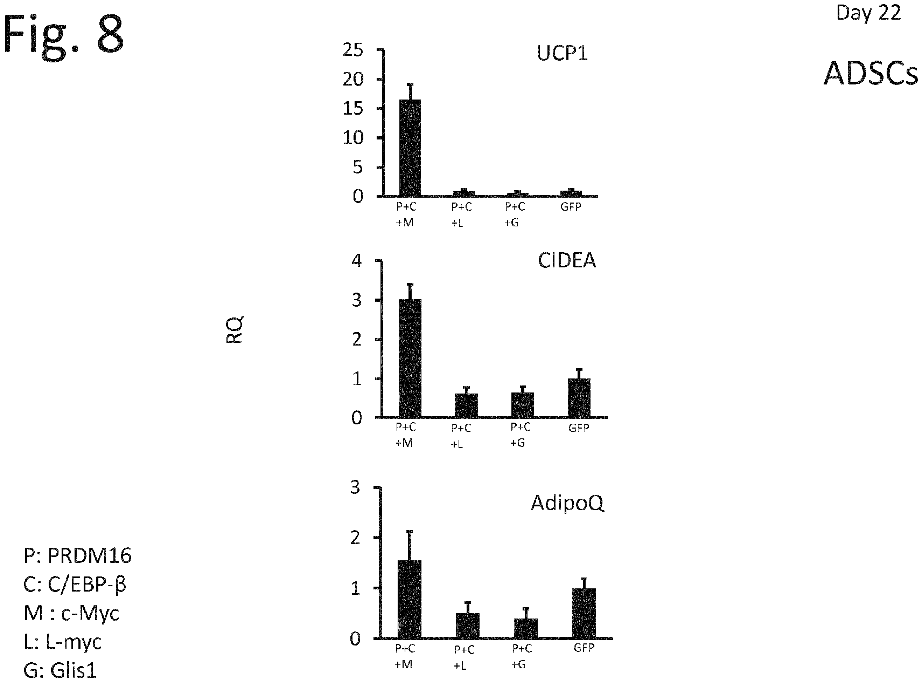

### Conversion from human adipose-derived stem cells into brown adipocytes, measurement of the mRNA expression of the UCP1 gene (Fig. 8)

Human adipose-derived stem cells (ADSCs) were cultured in 12-well plates, and an experiment was performed as shown in Fig. 1. Twenty-two days after gene transfer, total RNA was collected from the cells, and real-time RT-PCR was performed in the same manner as in Fig. 4 to quantify the mRNA expression of UCP1, CIDEA, and AdipoQ. Fig. 8 shows the results. Brown adipocyte-specific factors UCP1 and CIDEA were expressed at significantly higher levels in the group in which the three genes PRDM16, C/EBPβ, and c-Myc were introduced, compared to the group in which the three genes PRDM16, C/EBPβ, and L-Myc were introduced, the group in which the three genes PRDM16, C/EBPβ, Glis1 were introduced, and the control group in which the GFP gene was introduced. In contrast, adiponectin (AdipoQ), a marker common to brown adipocytes and white adipocytes, was expressed at a significantly high level in the group in which PRDM16, C/EBPβ, and c-Myc were introduced, and in the control group.

These results show that when human adipose-derived stem cells (ADSCs) are not transfected, many of them become white adipocytes; however, the introduction of the three genes PRDM16, C/EBPβ, and c-Myc allows for induction of brown adipocytes. This indicates that even cells other than normal human dermal fibroblasts can also be directly converted into brown adipocytes.

### Test Example 1

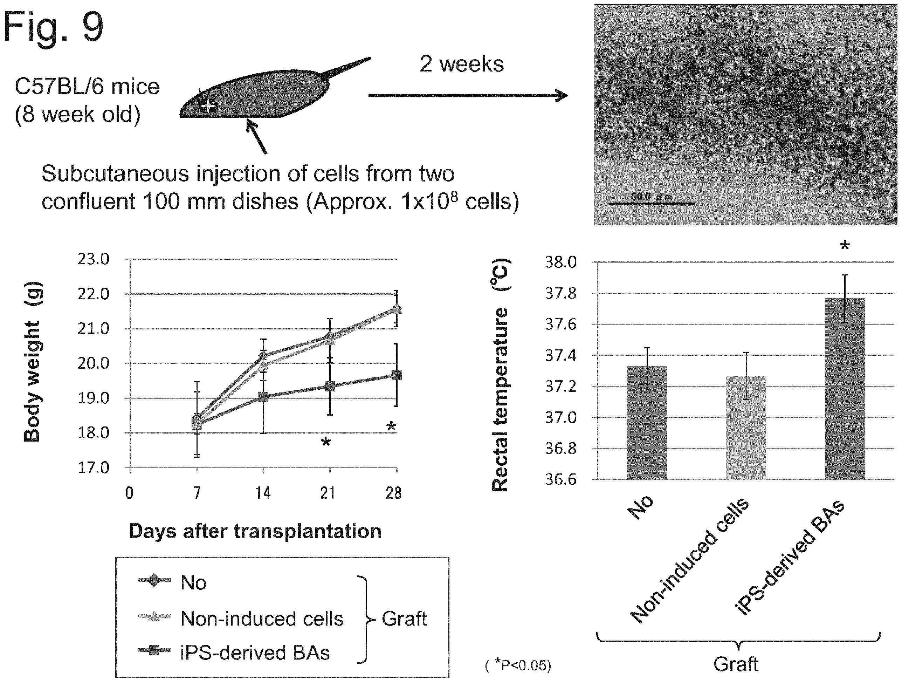

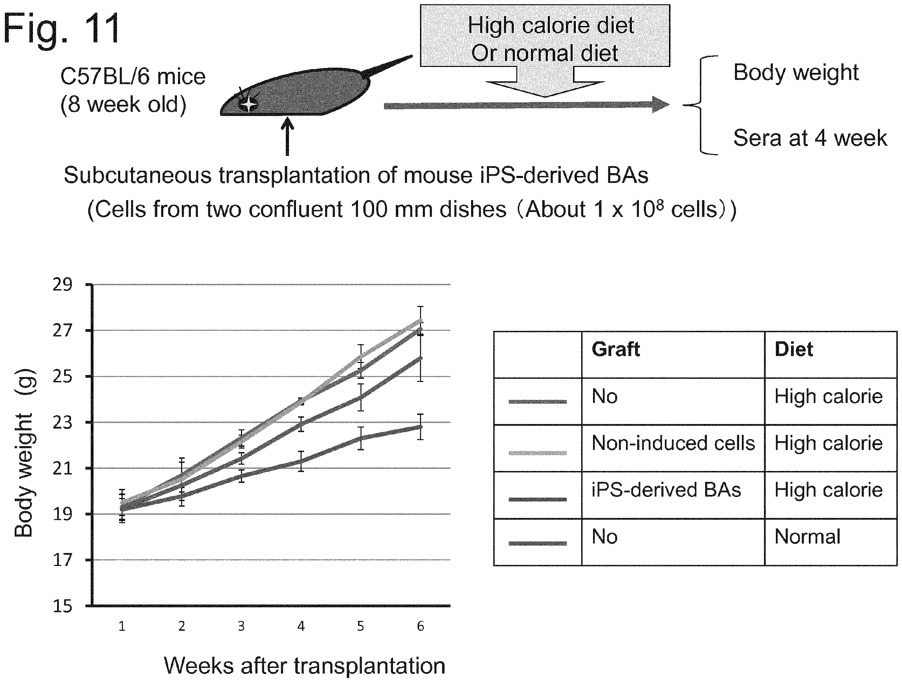

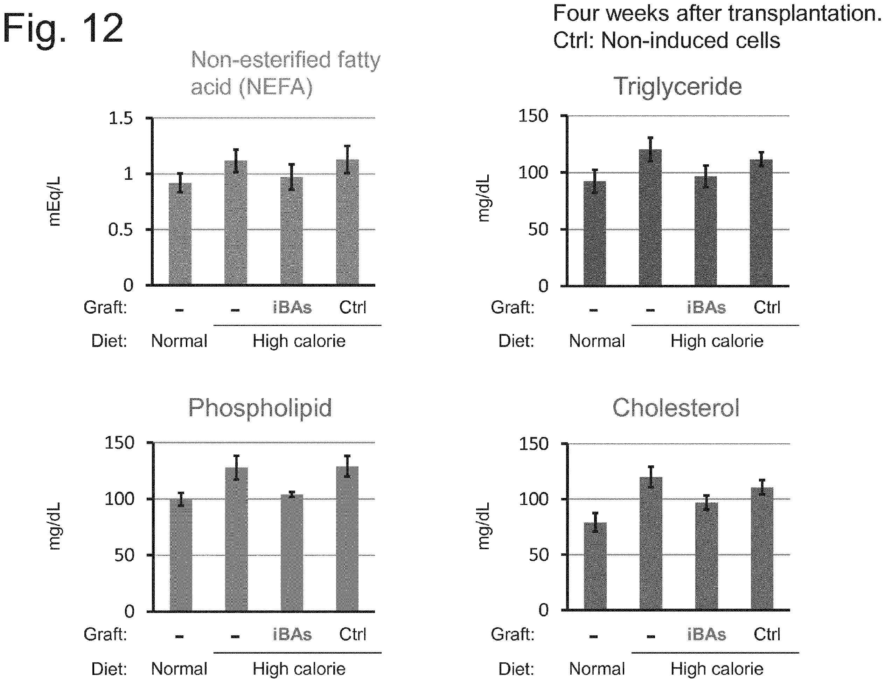

Figs. 9 to 12 show an experiment in which brown adipocytes were inducedfrom mouse iPS cells (iPS-derived BAs), and the brown adipocytes were transplanted into C57BL/6 mice. Thermogenesis and suppression in body weight increase were observed. Additionally, suppression in body weight increase and improvement in serum lipid abnormalities were observed in mice on a high-calorie diet. These results indicate that iPS-derived BAs have the potential to correct obesity and abnormal lipid metabolism associated with a high-calorie diet.

### (1) Fig. 9

The mouse iPS-derived BAs or non-induced cells as a control were transplanted under the abdominal skin of syngeneic mice as shown in the upper left of Fig. 9. The graft of the iPS-derived BAs exhibited an image of Oil Red O staining-positive adipose tissue (upper right). The mice into which the iPS-derived BAs were transplanted showed significant suppression in body weight increase (lower left) and significant elevation in body temperature (lower right).

### (2) Fig. 10

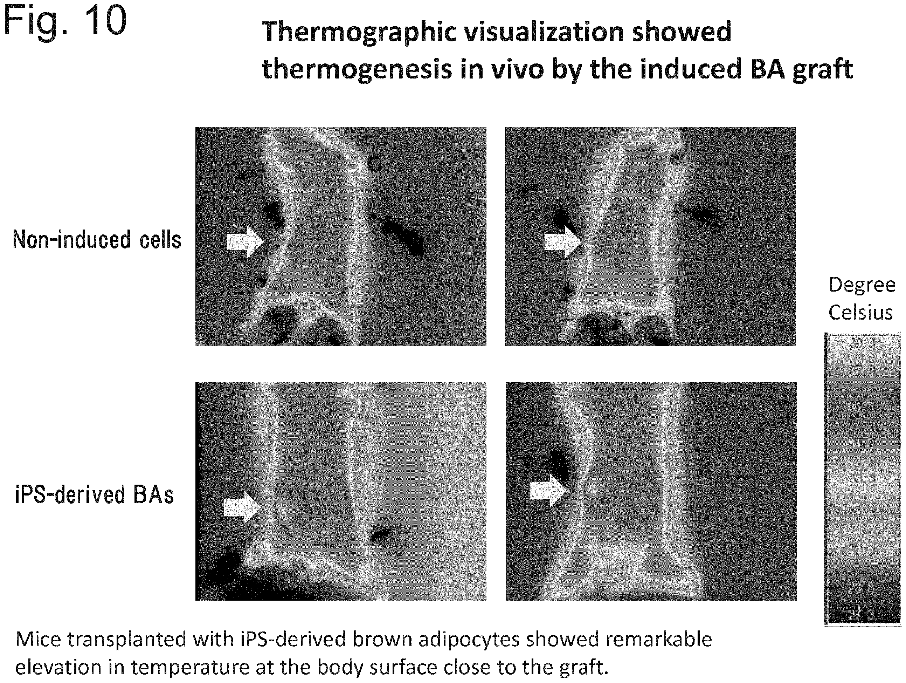

The mouse iPS-derived BAs or non-induced cells as a control were transplanted under the abdominal skin of syngeneic mice. Fig. 10 show thermographic images of two mice for each group. Elevation in temperature at the graft part of the iPS-derived BAs was observed (bottom). The graft of the cells that had not been induced into brown adipocyte had the same temperature as that of the surrounding tissue (top).

### (3) Fig. 11

After transplantation, the mice were fed a high-calorie diet. The results show that body weight increase was significantly suppressed only in the mice into which the iPS-derived BAs were transplanted.

### (4) Fig. 12

After transplantation, the mice were fed a high-calorie diet, and serum lipids were examined 4 weeks after transplantation. The results show that progression of hyperlipidemia was not observed in only the mice into which the iPS-derived BAs (iBAs) were transplanted. "Normal"indicates mice that were fed a normal diet.

### Test Example 2

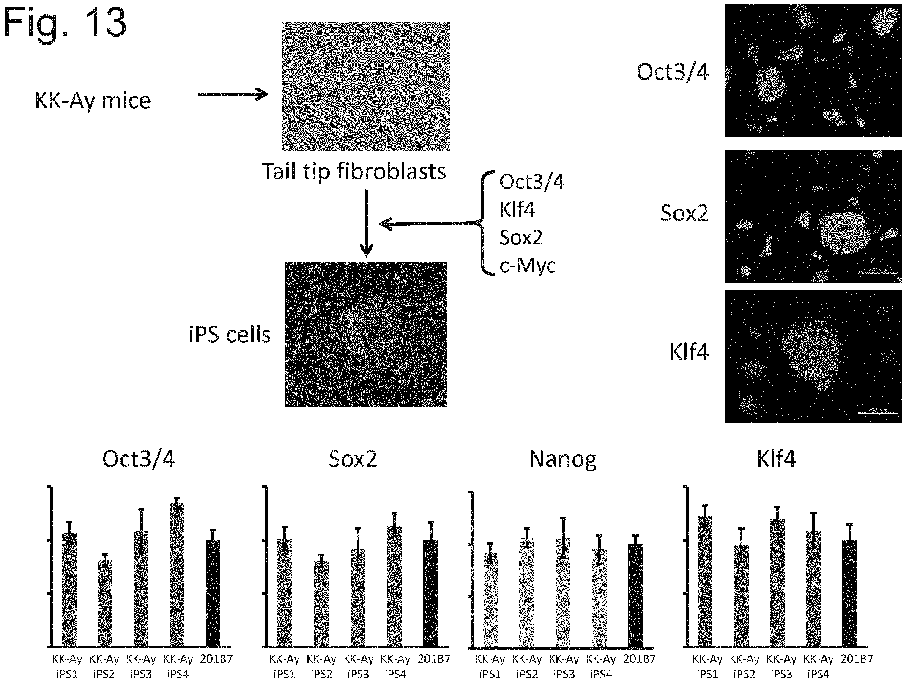

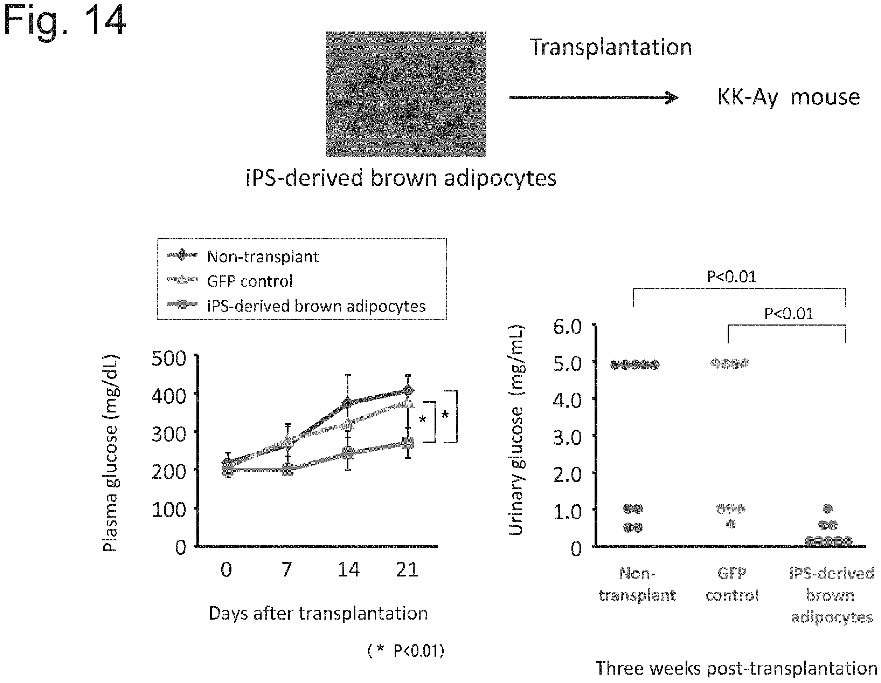

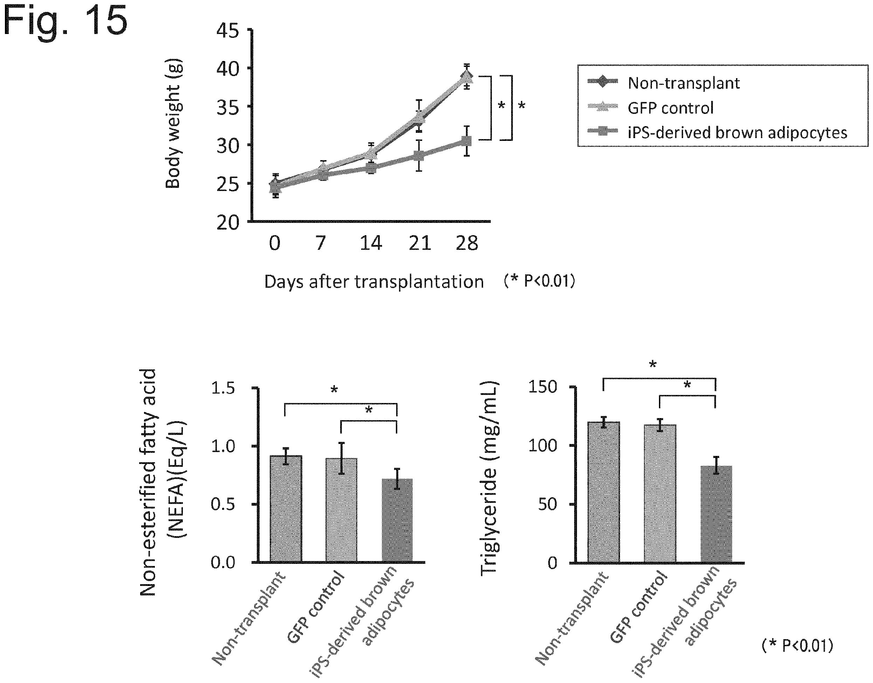

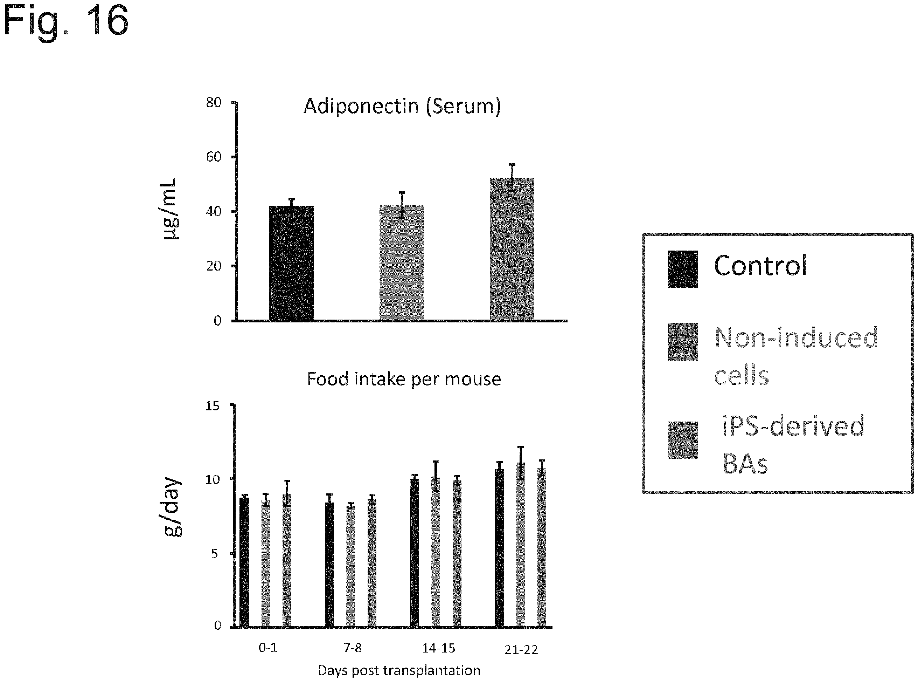

Figs. 13 to 16 show an experiment in which iPS cells were prepared from somatic cells of mice with type II diabetes, brown adipocytes were induced from the iPS cells (iPS-derived BAs), and the iPS-derived BAs were transplanted into diabetic mice. Reduction in non-fasting blood glucose levels, suppression in body weight increase, and improvement in serum lipid abnormalities were observed due to the transplantation. This shows that transplantation of brown adipocytes can control diabetes.

### (1) Fig. 13

iPS cells were prepared from somatic cells of KK-Ay mice, which develop type II diabetes. The obtained iPS cells showed typical ES-cell-like morphology (middle: a phase contrast microscope image) and expressed stem cell markers (bottom: real-time RT-PCR; right: immunofluorescence staining). KK-Ay iPS 1 to 4 are four different iPS cell clones derived from somatic cells of KK-Ay, whereas 201B7 is an iPS cell clone derived from normal mice.

### (2) Fig. 14

Brown adipocytes were induced from the iPS cells of Fig. 13 (iPS-derived BAs) (top). The iPS-derived BAs were transplanted into KK-Ay mice, and the results show that the increase in non-fasting blood glucose levels was moderate (lower left), and that almost no urinary glucose was detected (lower right). Progression of diabetes was observed in the controls, i.e., non-transplanted KK-Ay mice (Non-transplant), and mice transplanted with green fluorescent protein (GFP) gene-introduced cells that had not been induced into brown adipocytes (GFP control).

### (3) Fig. 15

The KK-Ay mice into which the iPS-derived BAs were transplanted showed significant suppression in body weight increase (top), and had low NEFA (lower left) and low neutral fat (lower right) in the serum.

### (4) Fig. 16

The KK-Ay mice into which the iPS-derived BAs were transplanted showed significantly high adiponectin in the serum. The amount of food intake was the same as that of the control mice.

### Example 9

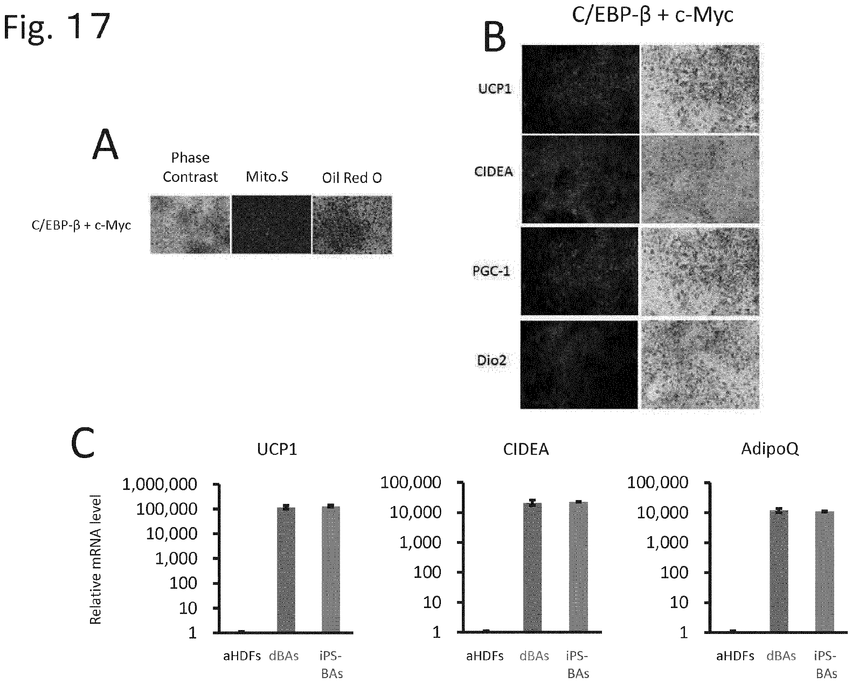

### Characteristics of brown adipocytes induced from normal human dermal fibroblasts and iPS cells (Fig. 17)

### A

Normal human dermal fibroblasts aHDFs were cultured in 12-well plates, and the two genes C/EBPβ and c-Myc were introduced thereinto by the method shown in Fig. 1. Fig. 17A shows the results of the thus-obtained group. Twelve days after gene transfer, the cells were observed with a phase contrast microscope (left). To visualize mitochondria, Mito Tracker Red probe produced by Invitrogen was added to the culture medium in some wells to a final concentration of 200 nM. The cells were allowed to stand in 5% CO₂/95% air at 37°C for 15 minutes, and then observed with a fluorescence microscope produced by Olympus Corporation (middle). The cells in some other wells were stained with Oil Red O in the same manner as in Fig. 2 (right). Numerous brown adipocytes containing numerous mitochondria accumulated therein and multilocular lipid droplets were observed.

### B

Normal human dermal fibroblasts aHDFs were cultured in 12-well plates, and an experiment was performed as shown in Fig. 1. Fig. 17B shows the results of a group in which the two genes C/EBPβ and c-Myc were introduced. Twelve days after gene transfer, immunostaining was carried out. In order from the top, the cells stained with anti-UCP1 antibody (primary antibody) and PE Cy5-labeled anti-rabbit IgG antibody (secondary antibody), the cells stained with anti-CIDEA antibody (primary antibody) and AlexaFluor488-labeled anti-rabbit IgG antibody (secondary antibody), the cells stained with anti-PGC-1 antibody (primary antibody) and PE Cy5-labeled anti-rabbit IgG antibody (secondary antibody), and the cells stained with anti-Dio2 antibody (primary antibody) and AlexaFluor488-labeled anti-rabbit IgG antibody (secondary antibody) are shown. The left images are fluorescent images, and the right images are differential interference images. All of the four proteins specific to brown adipocytes were shown to be expressed in large amounts.

### C

RNA was collected from normal human dermal fibroblasts aHDFs, brown adipocytes induced from aHDFs by the method of Fig. 17A (dBAs), and brown adipocytes induced from human iPS cells (iBAs), and real-time RT-PCR analysis was carried out using primers and probes specific to UCP1, CIDEA, and AdipoQ. Each mRNA expression amount was corrected by the amount of mRNA expression of β-actin, and relative mRNA expression amounts were calculated on the assumption that the mRNA expression amount in the aHDFs was 1. Fig. 17C shows the relative mRNA expression amounts. The dBAs and iBAs expressed the mRNA of brown adipocyte-specific genes at extremely high levels compared to the aHDFs.

### Example 10

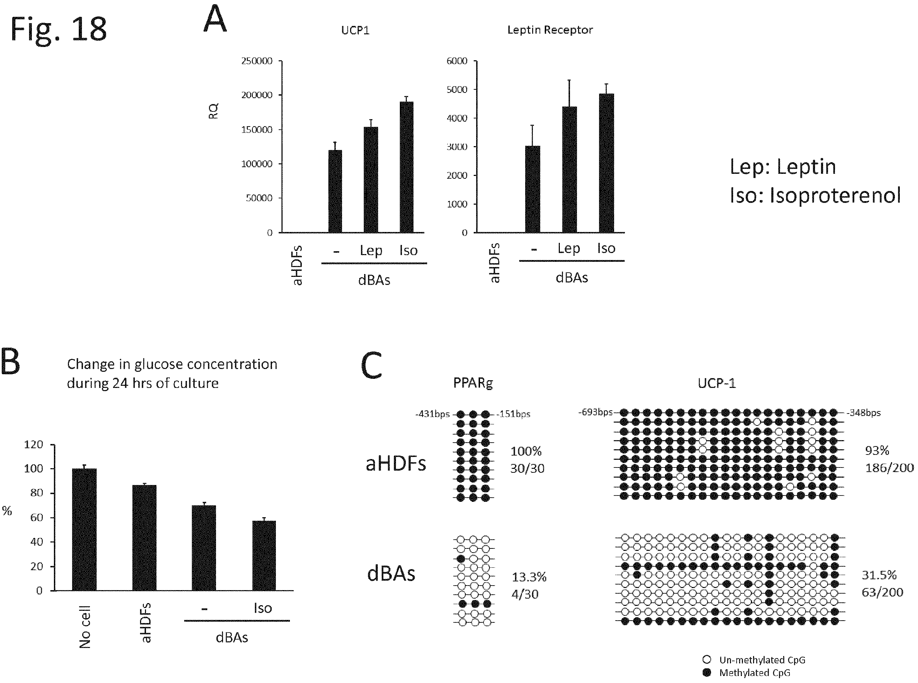

### Characteristics of brown adipocytes induced from normal human dermal fibroblasts (Fig. 18)

### A

RNA was collected from normal human dermal fibroblasts aHDFs, brown adipocytes induced from aHDFs by the method of Fig. 17A (dBAs), cells obtained by adding 100 nM of leptin to dBAs and culturing the dBAs for 24 hours (Lep), and cells obtained by adding 1 µM of isoproterenol to dBAs and culturing the dBAs for 2 hours (Iso). Real-time RT-PCR analysis was carried out using primers and probes specific to UCP1 and leptin receptor. Each mRNA expression amount was corrected by the amount of mRNA expression of β-actin, and relative mRNA expression amounts were calculated on the assumption that the mRNA expression amount in the aHDFs was 1. Fig. 18A shows the relative mRNA expression amounts. The dBAs expressed the mRNA of UCP1 and the mRNA of leptin receptor at extremely high levels compared to the aHDFs. These expression levels were shown to be further enhanced by stimulation of leptin or isoproterenol.

### B

Normal human dermal fibroblasts aHDFs, brown adipocytes induced from aHDFs by the method of Fig. 17A (dBAs), and cells obtained by adding 1 µM of isoproterenol to dBAs and culturing the dBAs for 2 hours (Iso) were prepared. The glucose concentration in each medium before and after culturing for 24 hours was measured with a glucose B test (Wako). Medium with no cells was incubated for 24 hours as a control. The percent of reduction in the glucose concentration in each medium was calculated. The results show that the dBAs consumed more glucose than the fibroblasts, and that the consumption was further enhanced by stimulation of isoproterenol.

### C

DNA was collected from normal human dermal fibroblasts aHDFs and brown adipocytes induced from aHDFs by the method of Fig. 17A (dBAs). Methylation of CpG dinucleotides of PPARg gene upstream region (-431 to -151 bp of the transcription start site) and UCP1 gene upstream region (-693 to -348 bp of the transcription start site) was analyzed by a bisulfite method. Methylation is shown in black, and demethylation is shown in white. The results reveal that the CpG dinucleotides in both regions were highly methylated in the fibroblasts, whereas hypomethylation was observed in the dBAs.

### Example 11

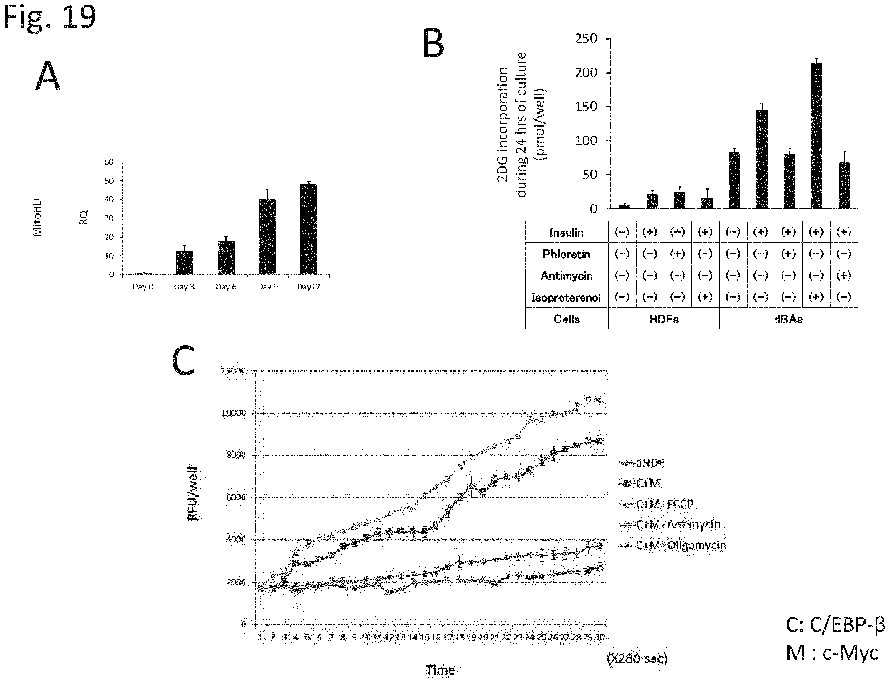

### Characteristics of brown adipocytes induced from normal human dermal fibroblasts (Fig. 19)

### A

An experiment for induction of brown adipocytes (dBAs) from normal human dermal fibroblasts aHDFs by the method of Fig. 17A was performed. RNA was collected from the cells on days 0, 3, 6, 9, and 12 after gene transfer, and real-time RT-PCR analysis was carried out using primers and a probe, each specific to MitoHD. Each mRNA expression amount was corrected by the amount of mRNA expression of β-actin, and relative mRNA expression amounts were calculated on the assumption that the mRNA expression amount in the aHDFs was 1. Fig. 19A shows the relative mRNA expression amounts. The results show that as induction of from the fibroblasts to the dBAs proceeded, the expression of MT-ND1 gene of mitochondrial DNA was enhanced.

### B

Normal human dermal fibroblasts aHDFs and brown adipocytes induced from aHDFs by the method of Fig. 17A (dBAs) were prepared. After the cells were plated in 6-well plates, insulin, phloretin, antimycin, and/or isoproterenol was added as shown in Fig. 19B, and incorporation of 2-deoxyglucose was measured. The results show that the dBAs exhibited high incorporation of glucose compared to the fibroblasts; this incorporation was increased by stimulation of insulin; and the increase in the incorporation of glucose due to stimulation of insulin was canceled by phloretin (glucose transporter inhibitor). It was also shown that the incorporation of glucose in the dBAs was increased by stimulation of isoproterenol, and that the increase in the incorporation of glucose due to stimulation of isoproterenol was canceled by antimycin (mitochondrial electron transport chain inhibitor).

### C

Normal human dermal fibroblasts aHDFs and brown adipocytes induced from aHDFs by the method of Fig. 17A (dBAs) were prepared. FCCP, antimycin, or oligomycin was added, and the extracellular oxygen concentration was measured over time. The vertical axis indicates phosphorescence intensity, showing that the higher the value, the lower the oxygen concentration. The results reveal that the oxygen consumption was high in the dBAs compared to the fibroblasts, and that the oxygen consumption was suppressed by addition of antimycin or oligomycin.

### Example 12

Brown adipocytes are induced from human iPS cells by the method of Fig. 17A. The obtained brown adipocytes contain multilocular lipid droplets and significantly express UCP1. Brown adipocytes are also induced from human white adipocytes by the method of Fig. 17A. The obtained brown adipocytes contain multilocular lipid droplets, and significantly express UCP1.

### Example 13

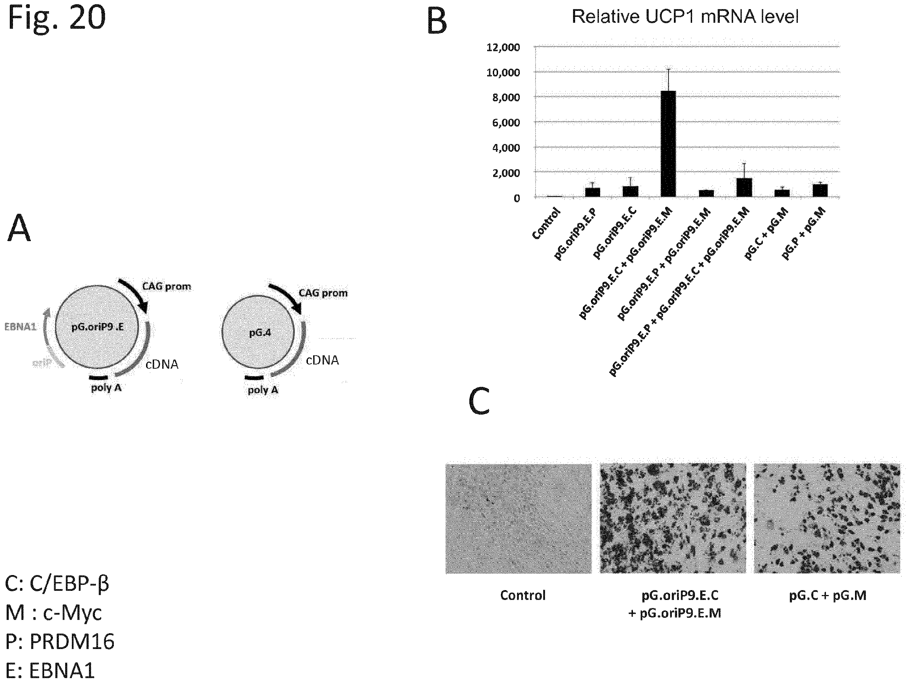

### Direct reprogramming of normal human dermal fibroblasts into brown adipocytes, using episomal vectors (Fig. 20)

### A

Fig. 20A shows the structures of an episomal vector and a plasmid vector. The episomal vector is shown on the left side of Fig. 20A. As cDNA of the episomal vector, a vector containing no genes is called pG.oriP9.E, a vector containing PRDM16 is called pG.oriP9.E.P, a vector containing CEBPbeta is called pG.oriP9.E.C, and a vector containing c-Myc is called pG.oriP9.E.M. The plasmid vector is shown on the right side in Fig. 20A. As cDNA of the plasmid vector, a vector containing no genes is called pG.4, a vector containing PRDM16 is called pG.P, a vector containing CEBPbeta is called pG.C, and a vector containing c-Myc is called pG.M. CAG prom indicates CAG promoter, polyA indicates Poly A additional signal, oriP indicates Epstein-Barr virus (EBV) origin of replication P, and EBNA1 indicates EBV nuclear antigen 1.

### B

After the plasmids of Fig. 20A were introduced into human skin-derived fibroblasts by electroporation, the cells were cultured for 12 days, and RNA was collected. Real-time RT-PCR was carried out using primers and a probe, each specific to the UCP1 gene. Each mRNA expression amount of the UCP1 gene was corrected by the amount of mRNA expression of β-actin, and relative mRNA expression amounts were calculated on the assumption that the mRNA expression amount in the fibroblasts was 1. Fig. 20B shows the relative mRNA expression amounts. The cells in which pG.oriP9.E.C and pG.oriP9.E.M were both introduced expressed UCP1 mRNA at the highest level, indicating induction of brown adipocytes.

### C

After the plasmids described above were introduced into human fibroblasts, the cells were cultured for 12 days. Fig. 20C shows images of Oil Red O staining. The results show that induction of fibroblast into brown adipocyte was efficiently induced by introducing both of two episomal vectors containing CEBPbeta and c-Myc, respectively (pG.oriP9.E.C and pG.oriP9.E.M). When both of two plasmid vectors containing CEBPbeta and c-Myc, respectively (pG.C and pG.M), are introduced, induction of fibroblasts into brown adipocytes can also be induced; however, the efficiency is inferior to that of the episomal vectors.

### Example 14

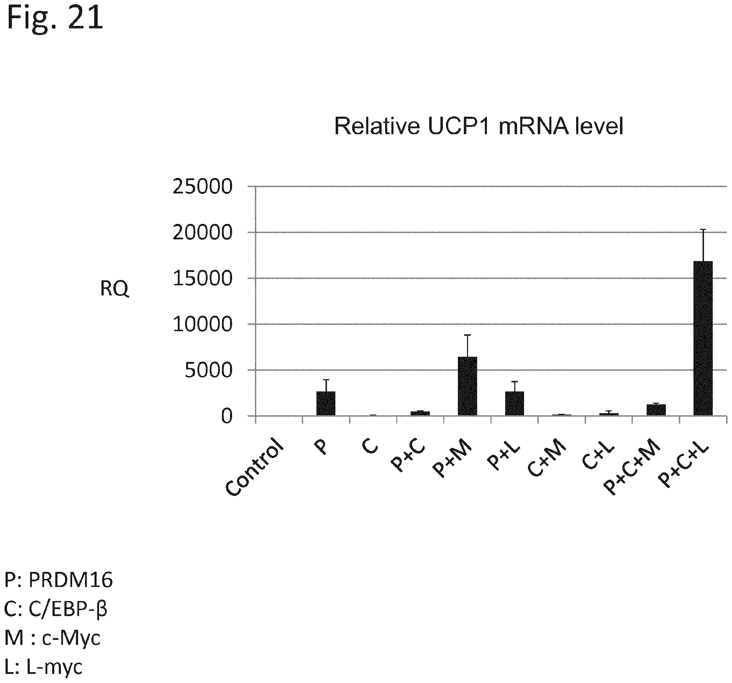

### Brown adipocytes induced from mouse embryonic fibroblasts (MEFs) (Fig. 21)

As in Example 1, retroviral vectors containing mouse PRDM16(P), C/EBPβ(C), L-Myc(L), and c-Myc(M) genes were used in various combinations to infect mouse embryonic fibroblasts (MEFs), and the cells were then cultured in brown fat induction medium. On day 20 after infection, RNA was collected. Mouse embryonic fibroblasts that were cultured in the same manner but not infected with retroviral vectors were used as a control. Real-time RT-PCR was carried out using primers and a probe, each specific to the UCP1 gene. Each mRNA expression amount of the UCP1 gene was corrected by the amount of mRNA expression of β-actin, and relative mRNA expression amounts were calculated on the assumption that the mRNA expression amount in the fibroblasts was 1. Fig. 21 shows the relative mRNA expression amounts. The cells in which the three factors PRDM16(P), C/EBPβ(C), and L-Myc(L) were introduced expressed UCP1 mRNA at the highest level, indicating induction of fibroblasts n into brown adipocytes.

### Example 15

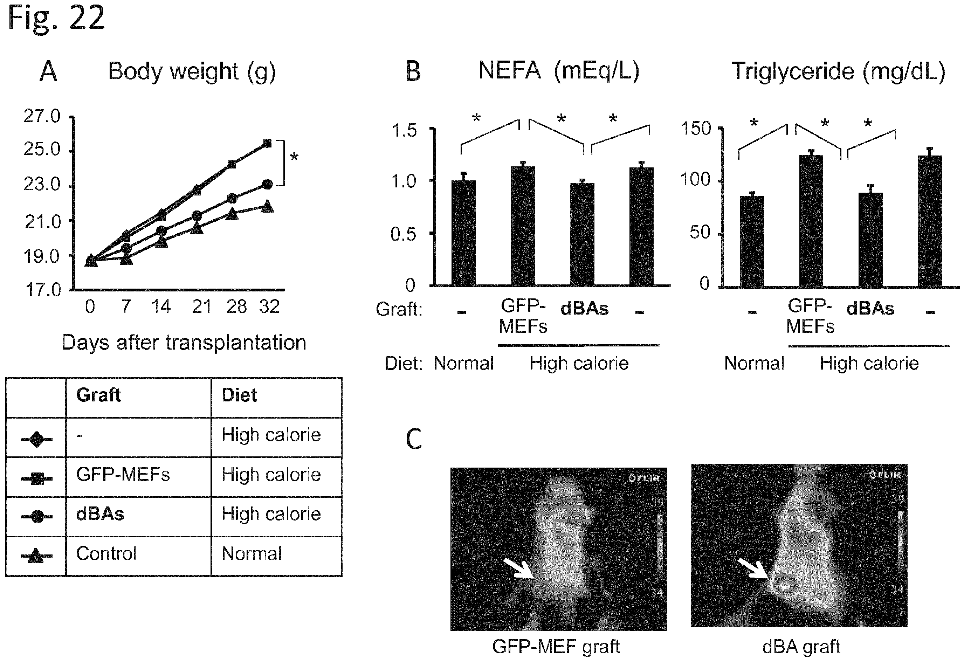

### Function in vivo of brown adipocytes induced from mouse embryonic fibroblasts (MEFs) (Fig. 22)

PRDM16(P), C/EBPβ(C), and L-Myc(L) genes were introduced into C57BL/6 mouse MEFs by the method of Example 13 to induce into brown adipocytes (dBAs). The dBAs or MEFs in which the GFP gene was introduced (GFP-MEFs) were subcutaneously transplanted into 8-week-old syngeneic mice. These mice and non-transplanted mice were fed a high-fat diet (high calorie). Non-transplanted mice fed a normal diet (normal) were prepared as a control.

### A

Fig. 22A shows the body weight of the mice after transplantation. The body weight of the non-transplanted mice and the GFP-MEFs-transplanted mice considerably increased with the intake of high calorie diet compared to the mice fed with normal diet, whereas diet-induced obesity was notably suppressed in the group into which the dBAs were transplanted.

### B

In the fourth week after transplantation, the serum was collected to measure non-esterified fatty acids (NEFA) and neutral fat (TG). The serum NEFA and TG in the non-transplanted mice and GFP-MEFs-transplanted mice considerably increased with the intake of high calorie diet compared to the mice fed with normal diet, whereas diet-induced dyslipidemia was notably suppressed in the group into which the dBAs were transplanted.

### C

On day 7 after transplantation, the GFP-MEFs-transplanted mice or the dBAs-transplanted mice were anesthetized and exposed to low temperature for 2 hours. Thereafter, the body surface temperature was measured by thermography. The body surface temperature at the transplanted site in the GFP-MEFs-transplanted mice was the same as the surrounding body surface temperature, whereas temperature elevation at the transplanted site was observed in the dBAs-transplanted mice.

### Example 16

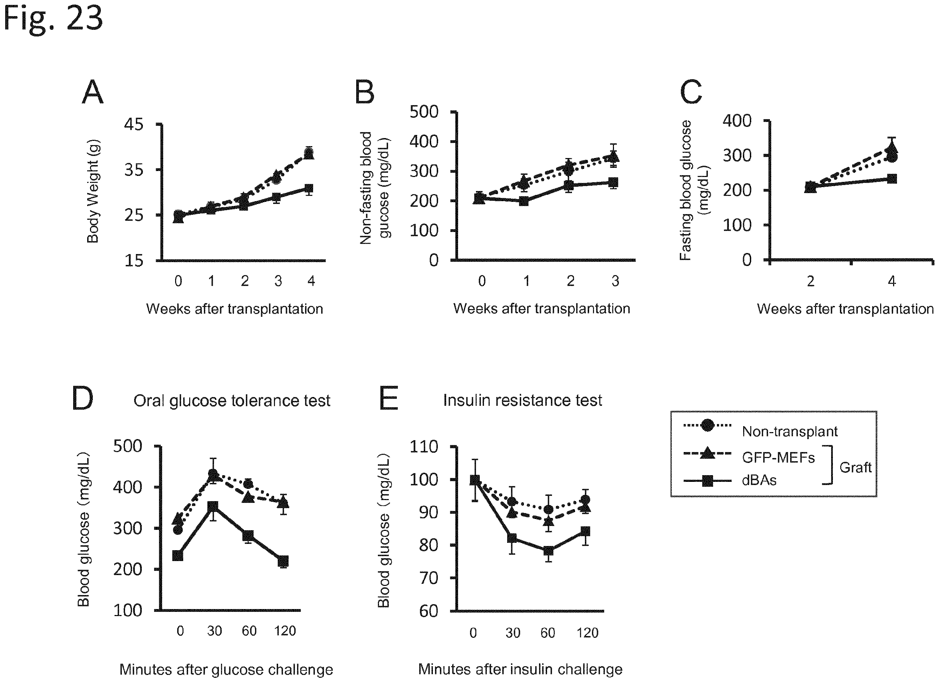

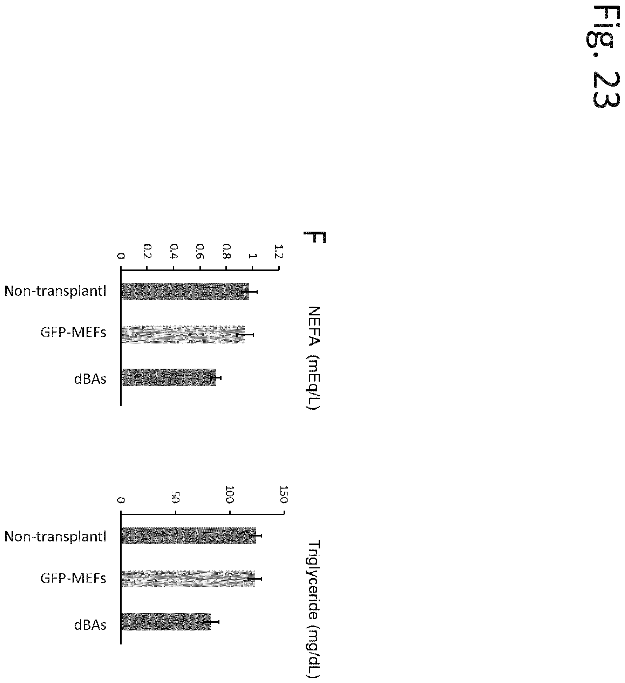

### Function in vivo of brown adipocytes induced from mouse embryonic fibroblasts (MEFs) (Fig. 23)

Brown adipocytes (dBAs) were induced from MEFs of diabetes model AAky mice by the method of Example 13, using PRDM16(P), C/EBPβ(C), and L-Myc(L) genes. The dBAs or MEFs in which the GFP gene was introduced (GFP-MEFs) were subcutaneously transplanted into 6-week-old syngeneic mice (10 cm Dish confluent x 2/mouse). These mice and non-transplanted mice were fed a normal diet.

### A-C

Fig. 23 shows the body weight of the mice (A), non-fasting blood glucose (B), and fasting blood glucose (C). It is shown that increase in body weight and elevation in blood glucose were significantly suppressed in the AAky mice into which the dBAs were transplanted.

### D

In the fourth week after transplantation, an oral glucose tolerance test was performed. Glucose was administered in an amount of 50 mg/mouse to the stomach of each mouse using a catheter, and blood glucose was measured over time. It is shown that glucose tolerance was improved in the mice into which the dBAs were transplanted.

### E

In the fourth week after transplantation, insulin resistance test was performed. Insulin was intraperitoneally injected into each mouse in an amount of 0.0125 U/mouse, and blood glucose was measured over time. It is shown that insulin resistance was improved in the mice into which the dBAs were transplanted.

### F

In the fourth week after transplantation, the serum of each mouse was collected to measure non-esterified fatty acids (NEFA) and neutral fat (TG). The serum NEFA and TG in the non-transplanted mice and GFP-MEFs-transplanted mice increased, whereas such dyslipidemia was notably suppressed in the group into which the dBAs were transplanted.

## Claims

1. An in vitro method for preparing a brown adipocyte from a somatic cell of a mammal by introducing at least one brown adipocyte-related gene or expression product thereof and at least one reprogramming-related gene or expression product thereof into the somatic cell, wherein a combination of the at least one brown adipocyte-related gene or expression product thereof and the at least one reprogramming-related gene or expression product thereof being any combination selected from the group consisting of CM, PCLMG, CLMG, PCLM, CLM, PCMG, PCM, and CMG, wherein P represents PRDM16, C represents C/EBPβ, L represents L-Myc, M represents c-Myc, and G represents Glis1, wherein brown adipocytes are directly induced from somatic cells

2. The method according to claim 1, wherein the somatic cell is a fibroblast or a white adipocyte.

3. A brown adipocyte-related gene or expression product thereof and a reprogramming-related gene or expression product thereof for use in the treatment of obesity, diabetes, impaired glucose tolerance, abnormal lipid metabolism, arteriosclerotic disease, hypertension, hyperuricemia, gout, non-alcoholic fatty liver disease or metabolic syndrome, wherein the treatment comprises converting a somatic cell in mammal into brown adipocyte directly, wherein a combination of the brown adipocyte-related gene and the reprogramming-related gene is any combination selected from the group consisting of CM, PCLMG, CLMG, PCLM, CLM, PCMG, PCM, and CMG, wherein P represents PRDM16, C represents C/EBPβ, L represents L-Myc, M represents c-Myc, and G represents Glis1.

## Patentansprüche

1. In-vitro Verfahren zum Herstellen eines braunen Adipozyten aus einer somatischen Zelle eines Säugetieres durch Einführen mindestens eines Genes, das auf braune Adipozyten bezogen ist, oder Expressionsproduktes davon und mindestens eines Genes, das auf Reprogrammierung bezogen ist, oder Expressionsproduktes davon in die somatische Zelle, wobei eine Kombination des mindestens einen Genes, das auf braune Adipozyten bezogen ist, oder Expressionsproduktes davon und des mindestens einen Genes, das auf Reprogrammierung bezogen ist, oder Expressionsproduktes davon irgendeine Kombination ausgewählt aus der Gruppe bestehend aus CM, PCLMG, CLMG, PCLM, CLM, PCMG, PCM und CMG ist, wobei P PRDM16 darstellt, C C/EBPβ darstellt, L L-Myc darstellt, M c-Myc darstellt und G Glis1 darstellt, wobei braune Adipozyten direkt aus somatischen Zellen induziert werden.

2. Verfahren nach Anspruch 1, wobei die somatische Zelle ein Fibroblast oder ein weißer Adipozyt ist.

3. Gen, das auf braune Adipozyten bezogen ist, oder Expressionsprodukt davon und ein Gen, das auf Reprogrammierung bezogen ist, oder Expressionsprodukt davon zur Verwendung in der Behandlung von Übergewicht, Diabetes, beeinträchtigter Glukosetoleranz, abnormalem Fettmetabolismus, arteriosklerotischer Krankheit, Bluthochdruck, Hyperurikämie, Gicht oder nichtalkoholbedingter Fettleberkrankheit, wobei die Behandlung das Umwandeln einer somatischen Zelle im Säugetier direkt in einen braunen Adipozyten umfasst, wobei eine Kombination des Genes, das auf braune Adipozyten bezogen ist, und des Genes, das auf Reprogrammierung bezogen ist, irgendeine Kombination ausgewählt aus der Gruppe bestehend aus CM, PCLMG, CLMG, PCLM, CLM, PCMG, PCM und CMG ist, wobei P PRDM16 darstellt, C C/EBPβ darstellt, L L-Myc darstellt, M c-Myc darstellt und G Glis1 darstellt.

## Revendications

1. Méthode *in vitro* de préparation d'un adipocyte brun à partir d'une cellule somatique d'un mammifère par introduction d'au moins un gène associé aux adipocytes bruns ou produit d'expression de celui-ci et d'au moins un gène associé à la reprogrammation ou produit d'expression de celui-ci à l'intérieur de la cellule somatique, dans laquelle une combinaison de l'au moins un gène associé aux adipocytes bruns ou produit d'expression de celui-ci et de l'au moins un gène associé à la reprogrammation ou produit d'expression de celui-ci étant toute combinaison sélectionnée dans le groupe constitué de CM, PCLMG, CLMG, PCLM, CLM, PCMG, PCM, et CMG, dans laquelle P représente PRDM16, C représente C/EBPβ, L représente L-Myc, M représente c-Myc, et G représente Glis1, dans laquelle les adipocytes bruns sont directement induits à partir de cellules somatiques.

2. Méthode selon la revendication 1, dans laquelle la cellule somatique est un fibroblaste ou un adipocyte blanc.

3. Gène associé aux adipocytes bruns ou produit d'expression de celui-ci et gène associé à la reprogrammation ou produit d'expression de celui-ci pour leur utilisation dans le traitement de l'obésité, du diabète, de l'intolérance au glucose, d'une anomalie du métabolisme lipidique, d'une maladie artériosclérotique, de l'hypertension, de l'hyperuricémie, de la goutte, de la stéatose hépatique non alcoolique ou du syndrome métabolique, dans lesquels le traitement comprend la conversion d'une cellule somatique chez un mammifère en un adipocyte brun directement, dans lesquels une combinaison du gène associé aux adipocytes bruns et du gène associé à la reprogrammation est toute combinaison sélectionnée dans le groupe constitué de CM, PCLMG, CLMG, PCLM, CLM, PCMG, PCM, et CMG, dans lesquels P représente PRDM16, C représente C/EBPβ, L représente L-Myc, M représente c-Myc, et G représente Glis1.
